# NEW EUROPEAN PATENT SPECIFICATION

(11) **EP 1 257 303 B2**
(45) Date of publication and mention of the opposition decision: **28.10.2009**
(45) Mention of the grant of the patent: 27.04.2005
(21) Application number: 01904880.0
(22) Date of filing: 17.01.2001
(51) Int. Cl.: A61L 15/36, A61F 13/15, B32B 9/00

(54) **ARTICLES COMPRISING LACTIC ACID PRODUCING MICROORGANISMS**
ARTIKEL DIE MILCHSÄUREPRODUZIERENDE MIKROORGANISMEN ENTHALTEN
ARTICLES CONTENANT DES MICRO-ORGANISMES PRODUISANT DE L'ACIDE LACTIQUE

(30) Priority: 18.01.2000 EP 00100261; 18.01.2000 EP 00100262; 18.01.2000 EP 00100257; 18.01.2000 EP 00100258
(43) Date of publication of application: 20.11.2002
(73) Proprietor: THE PROCTER & GAMBLE COMPANY, Cincinnati, Ohio 45202 (US)
(72) Inventor: DI CINTIO, Achille, I-65126 Pescara (IT); PESCE, Antonella, I-65120 Pescara (IT); CARLUCCI, Giovanni, I-66100 Chieti (IT); TALONE, Donatella, I-66043 Casoli (IT)
(74) Representative: Briatore, Andrea
(86) International application number: PCT/US2001/001464
(87) International publication number: WO 2001/052912

(56) References cited:
- EP-A- 0 811 392
- EP-A- 0 839 462
- WO-A-97/02846
- WO-A-99/17813
- WO-A-99/45099
- WO-A1-00//35502
- WO-A1-97//46194
- WO-A1-97//46195

## Description

### Field of the Invention

The present invention relates to absorbent articles in particular sanitary napkins and panty liners which combine the somehow contradictory benefit of high performing breathability and high protection level while delivering also effective malodour control benefits.

### Background of the Invention

The primary consumer needs which underlie development in the absorbent article field, in particular catamenials is the provision of products providing both a high protection and comfort level.

One means for providing consumer comfort benefits in absorbent articles is by the provision of breathable products. Breathability has typically concentrated on the incorporation of so called 'breathable backsheets' in the absorbent articles. Commonly utilized breathable backsheets are microporous films and apertured formed films having directional fluid transfer as disclosed in for example US 4 591 523. Both these types of breathable backsheets are vapour permeable allowing gaseous exchange with the environment. This thereby allows for the evaporation of a portion of the fluid stored in the core and increases the circulation of air within the absorbent article. The latter is particularly beneficial as it reduces the sticky feeling experienced by many wearers during use, commonly associated with the presence of an apertured formed film or film like topsheet, particularly over extended periods of time. This is a result of topsheets designed to achieve a clean and dry appearance. These topsheets tend to be smooth thereby minimizing the build up of fluid on the surface of the topsheet. However, these benefits are achieved at the expense of comfort, particularly under hot and humid conditions, when due to their smooth surface texture they tend to become sticky to the skin.

However, the main drawback associated with the use of breathable backsheets in absorbent articles is the negative effect on the protection level performance, by leakage known as wet through onto the users garment. Although, breathable backsheets in principle only allow the transfer of materials in the gaseous state, physical mechanisms such as extrusion, diffusion and capillary action may still occur and result in the transfer of the fluids from the absorbent core through the backsheet and onto the users garments. In particular, these mechanisms become more dominant if the article is utilized during physical exertion, or for heavy discharge loads or over extended periods of time.

Whilst the primary focus of absorbent articles remains the ability of these articles to be comfortable in use while absorbing and retaining fluids, another area of development is the control of odorous compounds within the articles.

Malodourous compounds typically present in absorbent articles originate from a number of sources. Firstly, the components of the fluid discharge such as urine, perspiration, menstrual fluid and blood may themselves contain malodorous compounds. Secondly, malodorous compounds may be generated as a result of the degradation of the components of the fluid discharge. Thus there are a wide range of compounds which may be present at some time during the use of an absorbent article which have an associated malodour.

It is believed that due to the very nature of a breathable absorbent article, malodorous compounds contained therein may, similar to air and vapour, be more readily exchanged with the environment. Hence, the malodourous compounds are able to escape from the article and are dissipated into the surroundings. Consequently, it is at least perceived by a number of potential users of absorbent articles that malodorous compounds are more easily detectable from breathable absorbent articles than from non breathable absorbent articles. The presence and detection of malodourous compounds from absorbent articles is however highly undesirable and may cause the wearer of these articles extreme embarrassment and thus, the prevention of their detection is highly desirable.

It is thus an object of the present invention to provide an absorbent article having a high comfort level together with a high level of protection and which reduces malodour perception by reducing the formation of malodour. It is yet a further object of the present invention to provide effective odour control over a broad range of malodors.

It has now been found that these objects are achieved by providing a breathable absorbent article, particularly by the provision of a breathable backsheet, which article comprises lactic-acid producing microorganisms. Indeed it has now surprisingly been found that the presence of lactic-acid producing microorganisms not only reduces or even prevents the formation of malodour but contribute at the same time to increase the level of protection by reducing the occurrence of leakage/wet through. Advantageously it has been found that one single ingredient used in an absorbent breathable article combines the dual benefit of malodour reduction over a broad range of malodors compounds and leakage/wet through reduction.

Indeed lactic acid producing microorganisms have the ability to deliver antagonistic properties against undesirable pathogens which are known to cause unpleasant odours like for example the bacteria belonging to the family Enterobacteriaceae, e.g., Proteus mirabilis, Proteus vulgaris, Echerichia coli and Klebsiella. The metabolic activity of such pathogen bacteria which aims for satisfying the bacteria needs for energy and proliferation, leads to degraded odorous compounds as by products which are among others low molecular weight fatty acids, amines, mercaptan, indoles, ammonia, sulfide and the like. The antagonistic microorganisms according to the present invention inhibit the growth of such pathogen microorganisms, by competing for substrate and generating a non conducive acidic environment by changing the pH to more acidic values (e.g., pH 4 to 4.5). The antagonistic properties of the lactic-acid producing microorganisms according to the present invention are also partly denoted their ability of producing other antimetabolites, like enzymes (e.g., lactoperoxidases), toxins, carbon dioxide, peroxides or antibiotics, so-called bacteriocines.

Additionally beside the odour formation prevention benefits other benefits are associated to the antagonistic microorganisms used herein. Indeed the competitive inhibition of pathogen microorganisms will also translate in reduction of the risk of infection in the urogenital environment of a user and/or in reduction of the risk of skin infection.

The present invention is further based on the finding that lactic acid producing microorganisms have the ability to influence the physical properties of bodily fluid discharge coming into contact therewith. Indeed a gelification of the bodily fluid is observed. Accordingly this translates in enhanced retention of the fluid in the absorbent product and thus in reduced leakage/wet through. Additionally the presence of such lactic-acid producing microorganisms in an absorbent article (e.g., in the absorbent core as described herein after in a preferred embodiment) reduces the rewetting of the fluid to the body which results in consumer noticeable dryness and cleanness benefit.

It is believed that the breathable environment does not only deliver the primary comfort benefit but also contributes to the effective odour prevention benefits associated with the articles according to the present invention. Indeed the breathability of the article, which reduces the hot, humid and anaerobic environment between the skin of the wearer and the surface of the absorbent article, contributes in an overall reduction of growth of microorganisms, thereby reducing the presence of pathogen organisms in the bodily fluid.

The reduction in the hot, humid and occlusive environment between the vicinity of the skin of the wearer and the article itself also reduces the tendency of the wearer to perspire. Consequently, the amount of associated perspiration related odour will be reduced. Thus, the breathability of the article actually reduces the amount of odour generated within the absorbent article.

An additional benefit of the present invention is that the breathability of the article further contributes to the dryness/cleanness benefit associated to the presence of the lactic acid producing microorganisms. Indeed the combination of breathability and the lactic acid producing microorganisms leads to an improvement in the overall dryness of the article. The breathability of the article allows for the evaporation of fluid from the article, and also as indicated above a reduction in the amount of perspiration generated by the wearer of the product and thus a reduction in hot and sweaty feelings often associated with the presence of topsheets designed to retain a clean and dry surface. The article therefore needs to retain less fluid and can do so more effectively. Furthermore the use of the breathable backsheet further contributes to a clean and dry facing surface (topsheet), such that the surface feels dry to the touch and the skin of the wearer of the article does not feel wet or moist and such that the wearer experiences minimal discomfort during wearing.

In a preferred embodiment the lactic-acid producing micro-organisms used herein are spore-forming lactic acid producing micro-organisms, preferably B. coagulans (also called herein Lactobacillus sporogenes). These microorganisms have the ability to create very quickly an environment that is not suitable for the growth of pathogens. This is due to the rapid growth, high yield and reproducibility of such micro-organisms in comparison to other lactic acid producing bacteria like Lactobacillus acidophilus. Furthermore these micro-organisms are spore forming micro-organisms, i.e., they are able to survive longer and reproduce themselves in comparison to non spore-forming microorganisms. In other words, by the formation of spores these microorganisms can germinate and re-germinate in time sequence in line with the bodily discharge (growth media) into an absorbent article, thereby ensuring long-lasting antagonistic properties against pathogens, typically long lasting odour control. Advantageously when using spore-forming lactic acid producing micro-organisms in the absorbent articles herein, the genetic identity, the lactic acid producing ability and viability of the microorganisms are maintained upon prolonged periods of use. Without to be bound by theory, it is speculated herein that in the event where the bodily fluid discharge is reduced and hence less substrate is available for the spore-forming micro-organisms, the micro-organisms used herein have the ability to be transformed in a dormant form (i.e., spore form), which will be activated again upon availability of further substrate, typically upon further bodily fluid discharge. Thus when providing the absorbent articles with such spore forming micro-organisms, effective antagonistic properties are delivered, e.g., odour controlling ability, while using reduced total amount of active material (typically odour control material).

In the most preferred embodiment of the present invention the dormant form of the spore-forming lactic acid producing micro-organisms, i.e., spores (preferably B. coagulans spores) are used as the lactic acid producing micro-organisms herein. These spores have the ability to germinate in the living form of the micro-organisms which exhibit antagonistic properties against undesirable pathogens. The use of such spores in the articles herein provides improved stability both during storage and use. Indeed the use of the spores herein provides an effective odour controlling article which can be stored for long periods of time before its actual use, without risking that the odour control ability of the article in use is impaired. Actually, the use of these spores able storage stability upon prolonged periods of time up to the time the article is used, i.e., is applied on the external surface of a human or animal body where it typically comes into contact with bodily fluids, and undergoes environmental changes that will create favourable environmental condition for the germination of the spores. Indeed the genetic identity, the lactic acid producing ability and the viability of the antagonistic microorganisms when using such spores are not impaired upon prolonged periods of storage and are maintained upon prolonged periods of use.

It is believed that the breathable environment contributes to the outstanding properties associated with the use of spores as described herein in absorbent articles according to the present invention. Indeed reduced perspiration will maintain the spores in their dormant form up to contact with bodily fluid discharge (menstruation, vaginal discharge and/or urine), and thus maintain the whole odour control capacity of the absorbent article up to the time bodily fluids contact the absorbent article. Thus effective odour control capacity is delivered, while using reduced total amount of active material, i.e., odour control material. Indeed a reduced total amount of spores according to the present invention is needed to obtain a given odour control activity for a given disposable article as compared to the total amount of non-spore forming antagonistic micro-organisms like Lactobacillus, Pediococcus, or Lactococcus that would be needed to get the same activity.

In a preferred embodiment herein the disposable breathable absorbent articles herein have an apertured polymeric film topsheet. This topsheet contributes to further improve the odor control benefit.

In a broadest aspect the present invention also encompasses articles, preferably a disposable absorbent articles, for controlling odours, said articles comprising lactic acid producing micro-organisms (preferably spore-forming lactic acid producing micro-organisms and/or spores thereof) and at least one odour absorbing agent. It has been surprisingly discovered that the combination of an odour absorbing agent, preferably silicate and/or zeolite, together with lactic acid producing micro-organisms in an article, like an absorbent article typically coming into contact with bodily fluids, results in a synergistic effect in terms of odour control. Indeed this combination gives more odour reduction than the odour reduction associated with the use of one of these two classes of ingredients alone at the same total level (either the odour absorbing agent alone or the lactic acid producing micro-organisms alone) in an absorbent article when in contact with bodily fluids.

Actually the combination of the lactic acid producing micro-organisms with an odour absorbing agent in an article herein allows to combine odour control mechanisms by which the overall malodour detection is synergistically reduced or even prevented.

In this broadest aspect of the invention the combination of the lactic acid producing micro-organisms with an odour absorbing agent allows odour control over a wide range of malodours which would otherwise not have been fully controlled by one of these two classes of ingredients used alone. More surprisingly, the presence of the lactic acid producing micro-organisms allows to increase the effectiveness of the odour absorbing agent. Indeed the lactic acid producing micro-organisms control the growth of pathogenic micro-organisms and as a consequence the amount of malodorous compounds produced by these micro-organisms. In other words, the lactic acid producing micro-organisms prevent the formation of malodour, thereby reducing the total amount of malodour to be controlled. This allows the odour absorbing agent to work in reduced amount of active. Actually this results in a more effective as well as a sustained use of the odour absorbing agent herein. Indeed the saturation point of the odour absorbing agent when used in association with the micro-organisms herein will be reached after prolonged periods of use, typically after prolonged wearing time of an absorbent article (pantiliner, pad) coming into contact with bodily fluid, as compared to when used alone in the same conditions. Advantageously it is believed that the odour absorbing agents also help the lactic-acid producing micro-organisms in reducing malodor by adsorbing the odor of head space (space between the absorbent article and the urogenital surface) and the malodorous components of fluids that are not controlled by these microorganisms.

### Background art of the invention

The incorporation of breathable backsheets in absorbent articles for improved wearer comfort has been described in the art such as for example in GB 2 184 389, US 3 881 489 and EP 203 821. EP-A 811 392 discloses breathable absorbent articles having a chelating agent based odor control system.

International Patent Application WO 92/13577 describes a tampon or sanitary napkin that has been impregnated with a culture of lactic-acid producing bacteria, preferably of the genus Pediococcus, isolated from healthy individuals. The tampon or sanitary napkin is intended for the prophylactic treatment of urogenital infections. WO 97/02846 discloses an absorbent article with antagonistic microorganisms selected from the family Lactobacillaceae, preferably from the genera Lactobacillus or Lactococcus. WO 98/47374 discloses articles of manufacture intended to be worn by or attached to skin or a mucous membrane of a mammal to allow probiotic activity of isolated Bacillus species to occur adjacent to or on the skin or mucous membrane. WO 92/13577, WO 97/02846 and WO 98/47374 all fail to disclose breathable absorbent articles.

None of these prior art references suggests the benefit of providing breathable absorbent articles comprising lactic acid producing microorganisms (preferably spore-forming lactic acid producing microorganisms), namely those of providing absorbent articles that combine high breathable performance for comfort together with reduced leakage/wet through while delivering effective odour control benefit over a broad range of malodours.

### Summary of the Invention

The present invention relates to an absorbent article, having a breathable backsheet and further comprising lactic acid producing microorganisms, preferably spore-forming lactic acid producing microorganisms and/or spores thereof.

The present invention also encompasses the use of lactic acid producing microorganisms (preferably spore-forming lactic acid producing microorganisms and/or spores thereof), in a breathable absorbent article, comprising a liquid permeable topsheet, an absorbent core and a breathable backsheet, for reducing leakage/wet through.

The present invention also encompasses the use of lactic acid producing microorganisms (preferably spore-forming lactic acid producing microorganisms and/or spores thereof), in an absorbent article, preferably a breathable absorbent article, comprising a wearing facing surface and a garment facing surface for improved dryness of the wearing facing surface.

In a broadest aspect the present invention relates to an article, preferably a disposable absorbent article, for controlling odours, preferably odours associated with bodily fluids, comprising lactic acid producing micro-organisms and at least one odour absorbing agent. In a preferred embodiment such article also comprises an absorbent gelling material.

### Detailed description of the Invention

By "article" it is meant herein any tridimentional solid material being able to receive/carry an odour control system as described herein comprising lactic acid producing micro-organisms and at least one odour absorbing agent. The term 'disposable' is used herein to describe articles which are not intended to be launched or otherwise restored or reused as an article (i.e., they are intended to be discarded after a single use and, preferably to be recycled, composted or otherwise disposed of in an environmentally compatible manner).

In the broadest aspect of the invention preferred articles include disposable absorbent articles that are designed to be worn in contact with the body of a user and to receive fluids discharged from the body, such as pantiliners, sanitary napkins, catamenials, incontinence inserts/pads, diapers, tampons, interlabial pads/inserts, breast pads, human or animal waste management devices and the like. Typically such human urine or faecal management devices comprise a bag having an aperture and a flange surrounding the aperture for preferably adhesive attachment to the urogential area and/or the perianal area of a wearer. Any faecal or urine management device known in the art is suitable for use herein. Such devices are described in for example WO 99/00084 to WO 99/00092. Other articles suitable according to the present invention also include other articles designed to be worn in contact with the body such as clothing, bandages, thermal pads, acne pads, cold pads, compresses, surgical pads/dressings and the like, articles for absorbing perspiration such as shoe insoles, shirt inserts, perspiration pads and the like, body cleansing articles like impregnated wipes/tissues (e.g. baby wipes, wipes for feminine intimate hygiene), and the like, and articles for animals like litters and the like.

By "bodily fluids" it is meant herein any fluid produced by human or animal body occurring naturally or accidentally like for instance in the case of skin cutting, including for instance perspiration, urine, menstrual fluids, faeces, vaginal secretions and the like.

The present invention primarily relates to breathable absorbent articles such as sanitary napkins, panty liners, incontinence devices, nursing pads/breast pads and baby diapers, interlabial pads. Typically such products comprise a liquid pervious topsheet, a backsheet and an absorbent core intermediate the topsheet and the backsheet. According to the present invention the breathability of the absorbent article is provided by the presence of a breathable backsheet which thereby allows the circulation of water vapour and preferably both water vapour and air through it. According to the present invention the absorbent article comprises lactic acid producing microorganisms, preferably B coagulans. It has now been found that the combination of the breathability of the absorbent article and lactic acid producing microorganisms results in a comfortable high performing breathable article with an unexpected improvement of the level of protection (i.e., reduced leakage/wet through) and a reduction or even prevention of the formation of malodors, when the article comes in contact with bodily fluids.

### Lactic acid producing microorganisms

According to the present invention the articles comprise as an essential component lactic acid producing microorganisms. It is understood herein that by lactic acid producing microorganisms reference is made to the use of one species thereof or mixtures thereof.

Suitable lactic acid producing microorganisms for use herein also called antagonistic microorganisms are microorganisms that exhibit antagonistic properties against undesirable strain of microorganisms by releasing amongst other metabolites, lactic acid. Suitable microorganisms that exhibit antagonistic properties for use herein include bacteria or other microorganisms for instance fungi.

Suitable lactic acid producing bacteria (antagonistic bacteria) for use herein include those belonging to the genera Lactobacillus, Lactococcus, Pedioccocus and/or Leuconostoc, and preferably the species Lactobacillus acidophilus, Lactobacillus curvatus, Lactobacillus plantarum, Lactobacillus jenseni, Lactobacillus casei, Lactobacillus fermentum, Lactococcus lactis, Pedioccocus acidilacti, Pedioccocus pentosaceus, Pedioccocus urinae and/or Leuconostoc mesenteroides.

Highly preferred lactic acid-producing microorganisms for use herein are the spore-forming lactic acid-producing microorganisms. These preferred microorganisms have the ability to survive in hostile environment in spore form (dormant form). Sporulation is the development in microorganisms of bodies each wrapped in a protective coat (a natural process of microencapsulation in a calcium-dipicolinic acid-peptidoglycan complex). Under favorable conditions, the spores germinate into viable bacilli (living form) and carry on their life activities. The spores/endospores suitable for use herein are heat-resistant, dehydrated resting cells that are formed intracellularly and contain a genome and all essential metabolic machinery. The spores are encased in a complex protective spore coat.

Suitable spore-forming lactic acid-producing microorganisms for use herein or spores thereof belong to the genus Bacillus and are typically selected from the group consisting of Bacillus coagulans (also called Lactobacillus sporogenes), Bacillus subtilis, Bacillus laterosporus and Bacillus laevolacticus. Other suitable spore-forming lactic acid producing micro-organisms for use herein and/or spores thereof are those belonging to the genus Sporolactobacillus, more particularly the species Sporolactobacillus inulinus. Highly preferred herein is the species B. coagulans (also called L.sporogenes) (living form) or B coagulans spores (dormant form).

L. sporogenes was first isolated from green malt and described in 1933 by L.M. Horowitz-Wlassowa and N.W. Nowotelnow. It was submitted as L. sporogenes in the fifth edition (1939) of 'Bergey's manual of Determinative Bacteriology' as well as mentioned in recognized scientific publication, Korean J. Appl. Microb. & Bioengin. (1985) 13:185-190, J. Pharmaceut. Soc. Korea (1977) vol XXIII, 1-Feb, 473-474. L. sporogenes was transferred to Bacillus coagulans in the seventh edition of 'Bergey's manual of Determinative Bacteriology' due to simplification in cataloguing. However in honour of the original discoverers the name L. sporogenes is used widely. Reference is also made to the taxonomical classification of Sporolactobacillus in L'integratore Nutrizionale 2 (1) 1999, Stabilita' di integratori con Sporolactobacillus, classificazione tassonomica by L. Marossi and all.

According to the Eighth Edition of Bergey's Manual of Determinative Bacteriology, "various spore-bearing rods which produce lactic acid, are facultative or aerobic and catalase positive, have generally and correctly been assigned to the genus Bacillus'. The characteristics of species B. coagulans as cited in 'Bergey's Manual of Determinative Bacteriology' (seventh edition) and other sources are "non pathogenic gram positive spore-forming bacteria (rods 0.9 by 3.0 to 5.0 micron size), aerobic to microaerophilic, producing L (+) lactic acid (dextrorotatory) in homofermentation conditions". species B. coagulans also release other metabolites like carbon dioxide, diacetyl, bacteriocins, lactoperoxidase. It has been isolated from natural sources, such as heat-treated soil samples inoculated into nutrient medium (Bergey's Manual of Systemic Bacteriology, Vol. 2, Sneath, P.H.A. et al., eds., Williams & Wilkins, Baltimore, MD, 1986)

Since B. coagulans exhibits characteristics typical of both genera Lactobacillus and Bacillus, its taxonomic position between the families Lactobacillaceae and Bacillaceae has often been discussed. This along with the fact that there is no universally accepted official classification leaves room for controversy in the nomenclature. More information about L.sporogenes (also called herein B. coagulans) is available from the commercial brochure 69/107, incorporated herein by reference, of Sochim International s.p.a. Milano, a supplier of the spore form of L. Sporogenes.

There are a variety of different bacillus species, including, but not limited to many different strains available through commercial and public sources, such as the American Tissue Culture Collection (ATCC). As already said some authors refer to Bacillus coagulans as L. sporogenes. Bacillus coagulans Hammer deposited as Lactobacillus sporogenes by Kabushiki Kaisha Naruse Fermentation Research Laboratory is commercially available under ATCC number 31284 (Internet information source: http://www.atcc.org/). Bacillus coagulans strains are further available as ATCC Accession Numbers 15949, 8038, 35670, 11369, 23498, 51232, 11014, 12245, 10545 and 7050. Bacillus subtilis strains are available as ATCC Accession Numbers 10783, 15818, 15819, 27505, 13542, 15575, 33234, 9943, 6051 a, 25369, 11838, 15811, 27370, 7003, 15563, 4944, 27689, 43223, 55033, 49822, 15561, 15562, 49760, 13933, 29056, 6537, 21359, 21360, 7067, 21394, 15244, 7060, 14593, 9799, 31002, 31003, 31004, 7480, 9858, 13407, 21554, 21555, 27328 and 31524. Bacillus laterosporus strains are available as ATCC Accession Numbers 6456, 6457, 29653, 9141, 533694, 31932 and 64, including Bacillus laterosporus BOD. Bacillus laevolacticus strains are available as ATCC Accession Numbers 23495, 23493, 23494, 23549 and 23492.

The spore forming lactic acid producing micro-organisms, especially B. coagulans, are preferred herein as they have the ability to create very quickly an environment that is not suitable for the growth of pathogens. This is due to the rapid growth, high yield and reproducibility of such micro-organisms in comparison to other lactic acid producing bacteria like Lactobacillus acidophilus. For example B. coagulans needs 30 minutes for one generation while L. acidophilus needs 80 minutes. Furthermore these micro-organisms are spore forming lactic acid producing micro-organisms, i.e., they are able to survive longer and reproduce themselves in comparison to non spore-forming micro-organisms. Indeed in contrast to non-spore forming bacteria like Lactobacillus acidophilus, these spore forming micro-organisms like B. coagulans are transformed in spores (dormant form) when the substrate is reduced (in absence or reduced amount of bodily fluid discharge) and germinate again upon further bodily fluid discharge, i.e., reproduce themselves again through the spore form. In other words, by the formation of spores these micro-organisms can germinate and re-germinate in time sequence in line with the bodily fluid discharge (growth media) into an absorbent article, thereby ensuring long-lasting antagonistic properties against pathogens and typically effective and long lasting odour control.

It is understood herein that the spore-forming lactic acid producing micro-organisms as described herein also include the spores (dormant form) of the micro-organisms (living form). Such spores are typically activated due to the presence of substrate, temperature increase and pH change. The optimum growth temperature range for the spores of B. coagulans is between 30°C and 50°C and the optimum pH range is from 5.0 to 6.5.

B. coagulans spores are ellipsoidal bodies measuring 0.9 to 1.2 by 1.0 to 1.7 microns. These spores are commercially available in a white to greyish powder form. B. coagulans (also called L. sporogenes) spores powder is commercially available under the name LACTOSPORE® from SABINSA CORPORATION (Sochim international s.p.a, Milano). 1 gram of LACTOSPORE® corresponds to 15*10⁹ spores and thus to 15*10⁹ cfu (colony forming unit) of B. coagulans (L. sporogenes).

In the embodiment of the present invention where the spores as described herein before are used, the articles can be stored for longer periods of time before their actual use and still contain their whole antagonistic capacity up to the time the articles are used. Indeed B. coagulans cells (in spore form) are protected from destruction by environmental factors by the naturally-present microencapsulation system, the spore coat. These spores are activated by environmental changes like pH and temperature changes and availability of substrate. For instance when the article is contacted with the skin or the urogenital zone of a wearer, the temperature increases (from room temperature to 30°C-35°C) and substrates like perspiration (pH 6), menstrual fluid discharge (pH 6.5), and/or urine discharge (pH 6.4) are provided. Such conditions will active the germination/re-germination of the spores. The spore coat imbibe water, swell and the increased water content will cause a rise in the metabolic rate of the sporulated bacilli. Outgrowths will begin to protrude from the spore-coats. The outgrown cells germinate and transform into viable vegetative cell (also called 'living form' herein). The living form begin to proliferate multiplying rapidly. These living forms continue their metabolic activities producing lactic acid and other metabolites which render the environmental non-conducive for the growth of harmful pathogenic micro-organisms. The germination process and more particularly the re-germination process (i.e., subsequent germination after the micro-organisms have been transformed into spores upon decrease of substrate availability) will be influenced by the body discharge in the article, leading to a somehow controlled germination/re-germination process on demand. In other words all the spores will not germinate with the same kinetic but the germination will be in relation to the body fluid discharge on the article. This will contribute to further sustain the effective odour control activity.

Typically the number of lactic acid producing microorganisms per article exceeds 10² cfu, preferably exceeds 10⁴ cfu, more preferably is between 10⁵ cfu to 10¹² cfu and most preferably from 10⁶ to 10¹⁰ cfu.

Pathogens bacteria that cause bad smell may belong to, for example, the families Enterobacteriaceae, Ascomycetes, Pseudomonadaceae and Micrococcaceae and the genus Streptococcus. Examples of species and genera are Pseudomonas, Candida albicans, Escherichia coli, Proteus mirabilis, Proteus vulgaris, Enterococcus, Klebsiella, Staphylococcus and Streptococcus.

The particularities of the antagonistic microorganisms herein is that they inhibit other microorganisms by competing for substrates, forming metabolites like lactic acid, enzymes like lacto-peroxidases, toxins, carbon dioxide, peroxides or antibiotics, so-called bacteriocines. They have the ability to sustain the growth and reduce the patogenicity of many pathogens like ones mentioned herein before and especially Proteus, Pseudomonas, Echerichia, Klebsiella, Enterococcus, Staphylococcus, Streptococcus and Candida.

It is speculated that the production of lactic acid lowers the pH to 4-5, thereby inhibiting the growth of putrefactive organisms like E.coli, which require a higher optimum pH (typically 6 to 7) for favourable growth conditions. Furthermore undissociated lactic acid has the tendency to penetrate the membrane of pathogens, lowering their intracellular pH and/or interfering with their metabolic processes such as oxidative phosphorylation, thereby inhibiting the growth of such pathogens.

Other metabolites further contribute to inhibit the growth of pathogens. For example carbon dioxide is believed to reduce membrane permeability. Hydrogen peroxide / Lactoperoxidase are believed to oxidise basic proteins and to destroy the "enzymes factories" (ribosomes) of pathogens. Bacteriocins are proteins or protein complexes with bactericidal activity. Indeed, bacteriocins have the ability to link to particular receptors on the cell wall of microorganisms, thereby affecting the functionality of the cell wall/membranes. Bacteriocins are also able to affect DNA-synthesis and protein synthesis.

The particularity of the antagonistic microorganisms herein is that they are naturally occurring microorganisms that are non-toxic and do not have any negative biological effect on humans.

One advantage afforded by the use of antagonistic microorganisms is that there is avoided an undesired selection pressure on the micro environment, such as favoring potential desease-promoting microorganisms and therewith the risk of developing pathogenic strains that are resistant to antibiotics and chemopharmaceutical preparations. Since the antimicrobial system is based on a natural, biological process, there is less risk of environmental ecological and toxic disturbances.

Advantageously it has now been found that the lactic acid producing microorganisms have the advantage of changing the physical properties of bodily fluid. Indeed a gelifrcation of the bodily fluid is obtained when the fluid comes into contact with the lactic acid producing microorganisms. This results in reduced leakage/wet through of the breathable absorbent articles. Without to be bound by theory, it is speculated that the cause of this gelification is the denaturation of the proteins. Indeed the microorganism release lactic acid through glycogen fermentation. As proteins are sensitive to pH, the presence of lactic acid causes the soluble proteins contained into the bodily fluid to turn into insoluble form. This creates a sort of tri-dimensional net of molecules trapping globules, minerals, fats which results in the so called gelification of the bodily fluid.

In a broadest aspect the present invention is based on the finding that the use of lactic acid producing microorganisms in an absorbent article comprising a garment facing surface and a wearer facing surface provides improved dryness of the wearer facing surface. By improved dryness reference is made to the same absorbent article in absence of any lactic-acid producing microorganism. As used herein the tospheet provides said wearing facing surface and the backsheet provides the garment facing surface. Improved dryness and cleanness is particularly noticed on the wearing facing surface in those embodiments wherein the lactic acid producing microorganisms are located in the absorbent core.

In a broadest aspect the present invention also encompasses an article, preferably a disposable absorbent article, for controlling odours, preferably odours associated with bodily fluids, comprising lactic acid producing micro-organisms and at least one odour absorbing agent. This combination of lactic acid producing micro-organisms and at least one odour absorbing agent is preferably used in breathable absorbent articles to further enhancing the odour control properties of such articles. It has now surprisingly been found that the lactic acid producing micro-organisms, preferably the spore-forming lactic acid producing micro-organisms and/or spores thereof (like B. coagulans and/or B coagulans spores) when together with odour absorbing agent as described herein after provide significant improved odour control capacity versus odour associated with bodily fluids. More particularly their combination results in a synergistic odour reduction, as compared to the odour reduction obtained for each of these two classes of odour controlling agents taken alone, when used at the same total level, in a same article (typically a disposable absorbent article) coming into contact with bodily fluids. Without to be bound by theory, it is speculated that the antagonistic micro-organisms herein reduce or even prevent the generation of malodours by blocking the microbial and/or enzymatic activity of pathogenic micro-organisms responsible for the formation of malodorous compounds. This allows the odour absorbing agent to work in reduced amount of active. Actually this results in a more effective as well as a sustained use of the odour absorbing agent herein. Indeed the saturation point of the odour absorbing agent when used in association with the micro-organisms herein will be reached after prolonged periods of use, typically after prolonged wearing time of an absorbent article (pantiliner, pad) coming into contact with bodily fluid, as compared to when used alone (in absence of the micro-organisms according to the present invention) in the same conditions. Advantageously it is believed that the odour absorbing agents also help the lactic-acid producing micro-organisms in reducing malodor by adsorbing the odor of head space (space between the absorbent article and the urogenital surface) and the malodorous components of bodily fluids that do not get into contact or are not controlled by the lactic-acid producing micro-organisms.

The lactic acid producing microorganisms (living form) are typically used in a freeze-dried form. Their isolation process follows known routine processes for the isolation of pure cultures. The isolated pure cultures are then typed according to known methods, e.g., API. The desired lactic acid producing bacteria are then cultivated in a fermentor in a manner known per se, are separated from the medium using a separator or a centrifuge, are freeze-dried in a manner known per se and ground to a fine powder. The bacterial concentrate in powder so obtained is then typically mixed with a fermentable carbohydrate, e.g., glucose, to a desired concentration. Such powder is then ready for use in the absorbent article. Alternatively in some case it is possible to add the living bacteria to the absorbent article and then carry out a freeze drying thereof. Another powder available form of the microorganisms herein is a lyophilized form.

In one embodiment herein, the articles are dried to a moisture content of less than 10%, preferably less than 5% and most preferably less than 1%, calculated as percentage of weight of the article.

### Growth of B. Coagulans

The growth of various bacillus species to form cell cultures, cell pastes and spore preparations is generally well known in the art. B coagulans growth described herein after can readily be used for the other bacillus species. B. coagulans is aerobic and facultative, it growths typically in nutrient broth, pH 5.7 to 6.8 containing up to 2% (by weight) NaCl, although neither NaCl nor KCl are required for growth. It is optimally grown at about 30°C to 55°C and the spores can withstand pasteurization. B coagulans can be grown in a variety of media, although it has been found that certain growth conditions produce a culture which yields a high level of sporulation. For example, sporulation is enhanced if the culture medium includes 10 milligrams per liter of manganese sulfate, yielding a ratio of spores (dormant form) to vegetative cells (living form) of about 80:20. In addition certain growth conditions produce a bacterial spore which contains a spectrum of metabolic enzymes particularly suited for the present invention, i.e. control of pathogen microorganisms generating malodour development. Although spores produced by these particular growth conditions are preferred, spores produced by any compatible growth conditions are suitable for producing a B coagulans useful in the present invention.

Suitable media for growth of B coagulans includes Nutristart 701, PDB (potato dextrose broth), TSB (tryptic soy broth) and NB (nutrient broth) all well known and available from a variety of sources. Media supplements containing enzymatic digests of poultry and fish tissue, and containing food yeast are particularly preferred. A preferred supplement produces a media containing at least 60% protein, and about complex carbohydrates and 6% lipids. Media can be obtained from a variety of commercials sources, notably DIFCO (Detroit MI), OXOID (Newark NJ) BBL (Cockeyesville MD) and Troy Biologicals (Troy MI).

A particularly suitable procedure for the preparation of B. Coagulans is as follows. B coagulans Hammer bacterium (e.g., ATCC# 31284) was inoculated and grown in nutrient broth containing 5g Peptone, 3 g Meat extract, 10-30 mg MnSO₄ and 1000 ml distilled water adjusted to pH 7.0, using a standard airlift fermentation vessel at 30°C. The range of MnSO₄ acceptable for sporulation is 1mg/l to 1g/l. The vegetative cells can be actively reproduce up to 65°C and the spores are stable up to 90°C. After fermentation, the B coagulans Hammer bacterial cells are collected using standard methods (e.g., filtration, centrifugation) and the collected cells and spores can be lyophilized, spray dried, air dried or frozen. As described herein, the supernatant from the cell culture can be collected and used as an extracellular agent secreted by B coagulans which has antimicrobial activity useful in a formulation of this invention. A typical yield from the above culture is about 100 to 150 billion cells/spores per gram before drying. Spores maintain at least 90% viability after drying when stored at room temperature for up to seven years, and thus the effective shelf life of a composition containing B coagulans Hammer spores at room temperature is about 10 years.

### Sources of B coagulans

Purified B coagulans bacterium are available from the American type Culture Collection (Rockville, MD) using the following accession numbers: B coagulans Hammer NRS T27 (ATCC# 11014), B coagulans Hammer strain C (ATCC# 11369), B coagulans Hammer (ATCC # 31284) and B coagulans Hammer NCA 4259 (ATCC# 15949). Purified B coagulans bacterium are also available from the Deutsche Sammlung con Miroorganismen und Zellkuturen GmbH (Braunschweig, Germany) using the following accession numbers: B coagulans Hammer 1915^{AL} (DSM#2356), B coagulans Hammer 1915^{AL} (DSM#2383, corresponds to ATCC#11014), B coagulans Hammer^{AL} (DSM#2384, corresponds to ATCC#11369) and B coagulans Hammer^{AL} (DSM#2385, corresponds to ATCC# 15949). B coagulans Bacterium can also be obtained from commercial suppliers such as Sabinsa Corporation (Piscataway,NJ) Sochim International s.p.a. (Milano, Italy).

These B coagulans strains and their growth requirements have been described previously (Baker et al, Can.J.Microbiol. 6:557-563, 1960, Blumenstock, "Bacillus coagulans Hammer 1915 und andere thermophile oder mesophile, sauretolerante bacillus-Arten-eine taxonomische Untersuchung' Doctoral thesis, Univ. Gottingen, 1984, Nakamura et al, Int. J. Syst. Bacteriol, 38:63-73, 1988). Strains of B coagulans can also be isolated from natural sources (e.g., heat-treated soil samples) using well known procedures (Bergey's Manual of Systemic Bacteriology, Vol. 2, p. 1117, Sneath, P.H.A. et al , eds, Williams &Wilkins, Baltimore, MD, 1986).

Further description of Bacillus species of interest, namely B coagulans and properties thereof can be found in WO 98/47374 to Ganeden Biotech. Inc, incorporated herein by references.

### Optional agents

The articles according to the present invention may further comprise on top of the lactic acid producing micro-organisms described herein before, other conventional agents or mixtures thereof.

### Absorbent gelling materials

As is well-known from recent commercial practice, absorbent gelling materials (sometimes referred to as "super-sorbers") are becoming broadly used in absorbent articles. AGM's are materials which have fluid-absorbing properties.

Such materials are highly preferred herein due to their dual function of absorbing and retaining fluids and odors, thereby further improving the level of protection and odor control.

Such materials form hydrogels on contact with water (e.g., with urine, blood, and the like). One highly preferred type of hydrogel-forming, absorbent gelling material is based on polyacids, especially polyacrylic acid. Hydrogel- forming polymeric materials of this type are those which, upon contact with fluids (i.e., liquids) such as water or body fluids, imbibe such fluids and thereby form hydrogels. These preferred absorbent gelling materials will generally comprise substantially water-insoluble, slightly cross-linked, partially neutralized, hydrogel-forming polymer materials prepared from polymerizable, unsaturated, acid-containing monomers. In such materials, the polymeric component formed from unsaturated, acid-containing monomers may comprise the entire gelling agent or may be grafted onto other types of polymer moieties such as starch or cellulose. Acrylic acid grafted starch materials are of this latter type. Thus, the preferred absorbent gelling materials include hydrolyzed acrylonitrile grafted starch, acrylic acid grafted starch, polyacrylates, maleic anhydride-based copolymers and combinations thereof. Especially preferred absorbent gelling materials are the polyacrylates and acrylic acid grafted starch.

Whatever the nature of the polymer components of the preferred absorbent gelling materials, such materials will in general be slightly cross-linked. Crosslinking serves to render these preferred hydrogel-forming absorbent materials substantially water-insoluble, and cross-linking also in part determines the gel volume and extractable polymer characteristics of the hydrogels formed therefrom. Suitable cross-linking agents are well known in the art and include, for example, (1) compounds having at least two polymerizable double bonds; (2) compounds having at least one polymerizable double bond and at least one functional group reactive with the acid-containing monomer material; (3) compounds having at least two functional groups reactive with the acid-containing monomer materials; and (4) polyvalent metal compounds which can from ionic cross-linkages. Cross-linking agents of the foregoing types are described in greater detail in Masuda et al; U.S. Patent 4,076,663; Issued February 28, 1978. Preferred cross-linking agents are the di- or polyesters of unsaturated mono-or polycarboxylic acids with polyols, the bisacrylamides and the di-or triallyl amines. Especially preferred cross-linking agents are N,N'-methylenebisacrylamide, trimethylol propane triacrylate and triallyl amine. The cross-linking agent will generally comprise from about 0.001 mole percent to 5 mole percent of the preferred materials. More preferably, the cross-linking agent will comprise from about 0.01 mole percent to 3 mole percent of the gelling materials used herein.

The preferred, slightly cross-linked, hydrogel-forming absorbent gelling materials will generally be employed in their partially neutralized form. For purposes described herein, such materials are considered partially neutralized when at least 25 mole percent, and preferably at least 50 mole percent of monomers used to form the polymer are acid group-containing monomers which have been neutralized with a salt-forming cation. Suitable salt-forming cations include alkali metal, ammonium, substituted ammonium and amines. This percentage of the total monomers utilized which are neutralized acid group-containing monomers is referred to as the "degree of neutralization". Typically, commercial absorbent gelling materials have a degree of neutralization somewhat less than 90%.

The preferred absorbent gelling materials used herein are those which have a relatively high capacity for imbibing fluids encountered in the absorbent articles; this capacity can be quantified by referencing the "gel volume" of said absorbent gelling materials. Gel volume can be defined in terms of the amount of synthetic urine absorbed by any given absorbent gelling agent buffer and is specified as grams of synthetic urine per gram of gelling agent.

Gel volume in synthetic urine (see Brandt, et al, below) can be determined by forming a suspension of about 0.1-0.2 parts of dried absorbent gelling material to be tested with about 20 parts of synthetic urine. This suspension is maintained at ambient temperature under gentle stirring for about 1 hour so that swelling equilibrium is attained. The gel volume (grams of synthetic urine per gram of absorbent gelling material) is then calculated from the weight fraction of the gelling agent in the suspension and the ratio of the liquid volume excluded from the formed hydrogel to the total volume of the suspension. The preferred absorbent gelling materials useful in this invention will have a gel volume of from about 20 to 70 grams, more preferably from about 30 to 60 grams, of synthetic urine per gram of absorbent gelling material.

Another feature of the most highly preferred absorbent gelling materials relates to the level of extractable polymer material present in said materials. Extractable polymer levels can be determined by contacting a sample of preferred absorbent gelling material with a synthetic urine solution for the substantial period of time (e.g., at least 16 hours) which is needed to reach extraction equilibrium, by then filtering the formed hydrogel from the supernatant liquid, and finally by then determining the polymer content of the filtrate. The particular procedure used to determine extractable polymer content of the preferred absorbent gelling agent buffers herein is set forth in Brandt, Goldman and Inglin; U.S. Patent 4,654,039; Issues March 31,1987, Reissue 32,649, The absorbent gelling materials which are especially useful in the absorbent articles herein are those which have an equilibrium extractables content in synthetic urine of no more than about 17%, preferably no more than about 10% by weight of the absorbent gelling material.

The absorbent gelling materials herein before described are typically used in the form of discrete particles. Such absorbent gelling materials can be of any desired shape, e.g., spherical or semi-spherical, cubic, rod-like polyhedral, etc. Shapes having a large greatest dimension/smallest dimension ratio, like needles and flakes, are also contemplated for use herein. Agglomerates of absorbent gelling material particles may also be used.

The size of the absorbent gelling material particles may vary over a wide range. For reason of industrial hygiene, average particle sizes smaller than about 30 microns are less desirable. Particles having a smallest dimension larger than about 2mm may also cause a feeling of grittyness in the absorbent article, which is undesirable from a consumer aesthetics standpoint. Furthermore, rate of fluid absorption can be affected by particle size. Larger particles have very much reduced rates of absorption. Preferred for use herein are absorbent gelling material s particles substantially all of which have a particle size of from about 30 microns to about 2mm. "Particle Size" as used herein means the weighted average of the smallest dimension of the individual particles.

Highly preferred absorbent gelling materials for use herein are partially neutralized absorbent gelling material (i.e., at least 25 mole percent of monomers used to form the polymer are acid group-containing monomers which have been neutralized with a salt forming cation). Indeed it has been found that the addition of absorbent gelling materials, especially partially neutralised absorbent gelling materials (e.g., cross linked sodium polyacrylate with a degree of neutralization of about 70% and pH of about 5.7 to 6.3 as XZ9589001 from Dow Chemicals) on top of the lactic acid producing microorganisms described herein in a disposable article according to the present invention results in further reduction of undesirable odours associated with bodily discharge occurrence in the article in use conditions. Without to be bound by any theory it is speculated that the absorbent gelling material, especially when partially neutralised, acts as a pH regulating substance (pH buffering agent), regulating the pH in a range from 4.5 to 6.8, preferably from 5 to 6.5), which is beneficial with respect to the inhibition of the growth of undesirable bacteria and therewith the occurrence of undesirable odours.

Typically, the amount of absorbent gelling material particles used in the articles herein, will range from 0 gm⁻² to 300gm⁻², preferably from 30gm⁻² to 150gm⁻², preferably from 30gm⁻² to 110gm⁻², more preferably from 55gm⁻² to 85gm⁻².

### Odour control agents

Additional odour control agent or combinations thereof, known in the art for this purpose may be used herein. These agents can typically be classified according to the type of odour the agent is intended to combat. Odors may be chemically classified as being acidic, basic or neutral.

Alternatively, the odor control agents may be categorized with respect to the mechanism by which the malodor detection is reduced or prevented. For example, odor control agents which chemically react with malodorous compounds or with compounds which produce malodorous degradation products thereby generating compounds lacking odor or having an odor acceptable to consumers may also be utilized herein.

Suitable odor control agents for use herein typically include carbonates (e.g., sodium carbonate), bicarbonates (e.g., sodium bicarbonate), phosphates (e.g., sodium phosphate), sulphates (e.g., zinc and copper sulphates), carboxylic acids such as citric acid, lauric acid, boric acid, adipic acid and maleic acid, activated carbons, clays, zeolites, silicas and starches. Such odor control agents and systems are disclosed in more details hereinafter and for example in EP-A-348 978, EP-A-510 619, WO 91/12029, WO 91/1197-7, WO 91/12030, WO 81/01643 and WO 96/06589. Alternative odor control agents are ion exchange resins such as those described in US 4 289 513 and US 3 340 875. Masking agents such as perfumes may also be used as odor control agents herein.

Typically, the articles herein may comprise the additional odor control agent or a mixture thereof at a level of from 0 gm⁻² to 600 gm⁻², preferably from 5 to 500 gm⁻², more preferably from 10 gm⁻² to 350 gm⁻² and most preferably from 20 gm⁻² to 200 gm⁻²

Preferred odour control agents for use herein are the odour absorbing agent or a mixture thereof. Indeed in a broadest aspect the present invention is based on a synergistic odour reduction by combining them with the micro-organisms as described herein. Suitable odour absorbing agents for use for this purpose include activated carbons, clays, zeolites, silicas, kieselguhr, starches, cyclodextrin, ion exchange resins and the like. Preferred herein are silicas and/or zeolites. Highly preferred herein is a mixture of silica and zeolite.

Cyclodextrin and derivatives thereof may be used as described in US 5 429 628. Ion exchange resins may be used such as those described in US 4 289 513 and US 3 340 875.

### Silica

Particularly suitable herein as an odor absorbing agent is silica. Silica, i.e. silicon dioxide SiO₂ exists in a variety of crystalline forms and amorphous modifications, any of which are suitable for use herein. In particular, silicas having a high surface area or in agglomerated form are preferred. Silica molecular sieves are not considered to be within the definition of silica as used herein. Preferably the silica is in a highly purified form such that is contains at least 90%, preferably 95%, more preferably 99% silicon dioxide. Most preferably the silica is silica gel having a 100% silica content. Alternatively, the silica may be provided from other sources such as metal silicates including sodium silicate.

According to the present invention the absorbent articles typically may comprise from 0 to 300 gm⁻², more preferably from 40 to 250 gm⁻² most preferably from 60 to 200 gm⁻², of silica based on 100% purity or a mixture thereof.

### Zeolite

Another particularly suitable odour absorbing agent herein is zeolite. The use and manufacture of zeolite material is well know in the literature and is described in the following reference texts: ZEOLITE SYNTHESIS, ACS Symposium Series 398, Eds. M. L. Occelli and H. E Robson (1989) pages 2-7; ZEOLITE MOLECULAR SIEVES, Structure, Chemistry and Use, by D. W. Breck, John Wiley and Sons (1974) pages 245-250, 313-314 and 348-352; MODERN APPLICATIONS OF MOLECULAR SIEVE ZEOLITES, Ph.D. Dissertation of S. M. Kuznicki, U. of Utah (1980), available from University of Microfilms International, Ann Arbor, Michigan, pages 2-8.

Zeolites are crystalline aluminosilicates of group IA and group IIA elements such as Na, K, Mn, Ca and are chemically represented by the empirical formula :

M_{2/n}O . Al₂O₃. ySiO₂ . wH₂O

where y is 2 or greater, n is the cation valence, and w is the water content in the voids of the zeolite.

Structurally, zeolites are complex, crystalline inorganic polymers based on an infinitely extending framework of AlO₄ and SiO₄ tetrahedra linked to each other by sharing of oxygen ions. This framework structure contains channels or interconnected voids that are occupied by the cations and water molecules.

The structural formula of a zeolite is based on the crystal unit cell, the smallest unit of structure, represented by

M_{x/n} [(AlO₂)ₓ (SiO₂)_{y}] . wH₂O

where n is the valence of cation M, w is the number of water molecules per unit cell, x and y are the total number of tedrahedra per unit cell, y/x usually having values of 1-5.

Zeolites may be naturally derived or synthetically manufactured. The synthetic zeolites being preferred for use herein. Suitable zeolites for use herein include zeolite A, zeolite P, zeolite Y, zeolite X, zeolite DAY, zeolite ZSM-5, or mixtures thereof. Most preferred is zeolite A.

According to the present invention the zeolite is preferably hydrophobic. This is typically achieved by increasing the molar ratio of the SiO₂ to AlO₂ content such that the ratio of x to y is at least 1, preferably from 1 to 500, most preferably from 1 to 6.

The articles typically comprise from 0 to 300 gm⁻², more preferably from 40 to 250 gm⁻² most preferably from 60 to 200 gm⁻², of zeolite based on 100% purity or a mixture thereof.

In a preferred embodiment herein the article comprises silica together with zeolite as the odour absorbing agents in a weight ratio of silica to zeolite in a range of from 1:5 to 5:1, preferably from 3:1 to 1:3 and most preferably about 1:1.

### Carbon material

The carbon material suitable for employment herein is the material well known in the art as an absorber for organic molecules and/or air purification purposes. Carbon suitable for use herein is available form a number of commercial sources under the trade names such as CALGON Type "CPG", Type SGL, Type "CAL" and type "OL". Often such material is referred to as "activated" carbon or "activated" charcoal. Typically it is available in the form of an extremely fine, dusty particles having large surface areas (200 - several thousand m²/g.) It is to be understood that any of the "air purifying" or "activated" carbons of commerce can be used in the practice of this invention.

### The disposable absorbent articles

Preferred breathable articles herein are pantiliners, feminine napkins, incontinent pads, diapers, nursing pads, and the like. The lactic acid producing microorganisms (and optional absorbing gelling material and/or optional additional odor control agent(s)) may be incorporated into an article by any of the methods disclosed in the art, for example layered on the core of the absorbent article or mixed within the fibres of the absorbent core.

The lactic acid producing microorganisms as described herein and optional additional agents are preferably incorporated between two layers of cellulose tissue. Optionally the system may be bonded between two cellulose tissue layers (laminate) with, for example, a hot melt adhesive (e.g., polyethylene powder) or any suitable bonding system like for instance glue like those commercially available from ATO Findley under the name H20-31® to glue the laminate layers and/or components together. Advantageously the use of conventional glue allows to avoid the heating step necessary when using polyethylene powder.

Adhesive lines are preferably also placed on the edges of the laminate to ensure that the edges of the laminate stick and any loose lactic acid producing microorganisms and optional additional agents present do not fall out of the laminate.

The lactic acid producing microorganisms may be distributed homogeneously or non homogeneously over the entire absorbent article or in at least one layer of the topsheet or in at least one layer of the backsheet or in at least one layer of the core or any mixture thereof. The lactic acid producing microorganisms may be distributed homogeneously or non homogeneously on the whole surface of the desired layer or layers, or on one or several area of the surface layer/layers to which it is positioned (e.g. central area and/or surrounding area like the edges of a layer of the absorbent article) or mixtures thereof. Preferably the lactic acid producing microorganisms are located in the core (also called intermediate layer which is positioned between the topsheet and the backsheet). The presence of the lactic acid producing microorganisms in the core is preferred because the core collects and absorbs bodily fluids. Thus the close proximity to the substrate contributes to improved antagonistic activity of the lactic acid producing microorganisms according to the present invention. Indeed optimum inhibition of the degradation activity by putrefactive bacteria and proliferation of pathogens is obtained as well as optimum leakage/wet through prevention by retaining the fluid in the core by the gelification/coagulation mechanism.

In one embodiment of the present invention the lactic acid producing microorganisms are positioned such that at least a portion of the fluid discharge comes into contact with said lactic acid producing microorganisms before the optional absorbent gelling material (e.g., AGM) and/or optional additional odor control agent if present. In particular, the lactic acid producing microorganisms can be located in a separate layer from the optional absorbing gelling material and/or optional additional odor control agent if present. In such an embodiment the lactic acid producing microorganisms are located towards the topsheet or in the topsheet itself (preferably the secondary topsheet) and the optional absorbing gelling material and/or optional additional odor control agent are located further away from the topsheet than the lactic acid producing microorganisms. In one embodiment of the present invention, the lactic acid producing microorganisms are positioned in at least one of the topsheet layers and the optional absorbing gelling material and optional additional odor control agent, if present, are positioned in the core. The benefits of these executions are related with the fact that the inhibitory action by microorganisms described herein starts immediately after the contact with body fluids and continue in the fluids storage layer. In this way pathogens microorganisms causing malodor have lower possibility and time to degrade body fluids already colonized by spores/microorganism as described herein typically B. coagulans.

In another embodiment the lactic acid producing microorganisms may be located in various layers, i.e. that the total amount of lactic acid producing microorganisms is distributed in the topsheet layer and the core. The benefit of this execution are related with the combined action of lactic acid producing microorganisms which inhibit the growth of putrefactive/pathogen bacteria in the core and on the body surface in contact with the absorbent article.

The lactic acid producing microorganisms as described herein may be incorporated as a powder or a granulate. When used in a granulate or particulate form the lactic acid producing microorganisms as described herein and the optional absorbing gelling material and optional odor control agent may be granulated separately and then mixed together or granulated together.

Suitable breathable absorbent article according to the present invention include those described as follows:

### Backsheet

According to the present invention, the absorbent articles comprise as an essential component a breathable backsheet. The primary role of the breathable backsheet is to prevent the extrudes absorbed and contained in the absorbent article from wetting articles that contact the absorbent article such as pyjamas and undergarments. In order to achieve this the backsheet typically extends across the whole of the absorbent structure and may extend into and form part of or all of sideflaps, side wrapping elements or wings. In addition to the prevention of liquid transport through the backsheet however, the breathable backsheet also permits the transfer of water vapour and preferably both water vapour and air through it and thus allows the circulation of air into and out of the backsheet and the absorbent article itself.

Suitable breathable backsheets for use herein include all breathable backsheets known in the art. In principle there are two types of breathable backsheets, single layer breathable backsheets which are breathable and impervious to liquids and backsheets having at least two layers, which in combination provide both breathability and liquid imperviousness.

Suitable single layer breathable backsheets for use herein include those described for example in GB A 2184 389, GB A 2184 390, GB A 2184 391, US 4 591 523, US 3 989 867, US 3 156 242 and European Patent Application number 95120653.1.

Suitable dual or multi layer breathable backsheets for use herein include those exemplified in US 3 881 489, US 4 341 216, US 4 713 068, US 4 818 600, EPO 203 821, EPO 710 471, EPO 710 472, European Patent Application numbers 95120647.3, 95120652.3, 95120653.1 and 96830097.0.

Particularly preferred are backsheets meeting the requirements as defined in European Patent Application number 96830343.8 and more preferably wherein the absorbent article also meets the requirements as described therein.

According to the present invention the breathable backsheet comprises at least one, preferably at least two water vapour permeable layers. Suitable water vapour permeable layers include 2 dimensional, planar micro and macro-porous films, monolithic films, macroscopically expanded films and formed apertured films. According to the present invention the apertures in said layer may be of any configuration, but are preferably spherical or oblong. The apertures may also be of varying dimensions. In a preferred embodiment the apertures are preferably evenly distributed across the entire surface of the layer, however layers having only certain regions of the surface having apertures is also envisioned.

2-dimensional planar films as used herein have apertures having an average diameter of from 5 micrometers to 200 micrometers. Typically, 2-dimensional planar micro porous films suitable for use herein have apertures having average diameters of from 150 micrometers to 5 micrometers, preferably from 120 micrometers to 10 micrometers, most preferably from 90 micrometers to 15 micrometers. Typical 2 dimensional planar macroporous films have apertures having average diameters of from 200 micrometers to 90 micrometers. Macroscopically expanded films and formed apertured films suitable for use herein typically have apertures having diameters from 100 micrometers to 500 micrometers. Embodiments according to the present invention wherein the backsheet comprises a macroscopically expanded film or an apertured formed film, the backsheet will typically have an open area of more than 5%, preferably from 10% to 35% of the total backsheet surface area.

Suitable 2-dimensional planar layers of the backsheet may be made of any material known in the art, but are preferably manufactured from commonly available polymeric materials. Suitable materials are for example GORE-TEX (TM) or Sympatex (TM) type materials well known in the art for their application in so-called breathable clothing. Other suitable materials include XMP-1001 of Minnesota Mining and Manufacturing Company, St. Paul, Minnesota, USA. As used herein the term 2 dimensional planar layer refers to layers having a depth of less than 1 mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition, the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at their terminating ends. Preferably said protuberances are of a funnel shape, similar to those described in US 3,929,135. The apertures located within the plane and the orifices located at the terminating end of protuberance themselves maybe circular or non circular, provided the cross sectional dimension or area of the orifice at the termination of the protuberance is smaller than the cross sectional dimension or area of the aperture located within the garment facing surface of the layer. Preferably said apertured preformed films are uni directional such that they have at least substantially, if not complete one directional fluid transport towards the core. Suitable macroscopically expanded films for use herein include films as described in for example in US 637 819 and US 4,591,523.

Suitable macroscopically expanded films for use herein include films as described in for example US 4 637 819 and US 4 591 523.

Suitable monolithic films include Hytrel™, available from DuPont Corporation, USA, and other such materials as described in Index 93 Congress, Session 7A "Adding value to Nonwovens", J-C. Cardinal and Y. Trouilhet, DuPont de Nemours International S.A., Switzerland.

According to the present invention the backsheet may comprise in addition to said water vapour permeable layer additional backsheet layers. Said additional layers may be located on either side of said water vapour permeable layer of the backsheet. The additional layers may be of any material, such as fibrous layers or additional water vapour permeable layers as described herein above.

In a particularly preferred embodiment herein a dual or multiple layer breathable backsheet composite is used in the absorbent article. According to the present invention suitable breathable backsheets for use herein comprise at least a first and a second layer. The first layer is positioned between the garment facing surface of the absorbent core and the wearer facing surface of the second layer. It is oriented such that it retards or prevents liquid from passing from the absorbent core towards the outside while allowing free air flow and water vapor through it. The second layer provides water vapor and air permeability so as to support breathability of the article. In addition to water vapor permeability the air permeability is desirable in order to further improve the comfort benefit from the breathability of the article.

Such a first layer is preferably in direct contact with the absorbent core. It provides air and water vapor permeability by being apertured. Preferably this layer is made in accordance with the aforementioned US-A-5,591,510 or PCT WO-97/03818, WO-97/03795. In particular, this layer comprises a polymeric film having capillaries. The capillaries extend away from the wearer facing surface of film at an angle which is less then 90 degrees. Preferably the capillaries are evenly distributed across the entire surface of the layer, and are all identical. However, layers having only certain regions of the surface provided with apertures, for example only an area outside the region aligned with the central loading zone of the absorbent core, maybe provided with such capillaries.

Methods for making such three-dimensional polymeric films with capillary apertures are identical or similar to those found in the apertured film topsheet references, the apertured formed film references and the micro-/macroscopically expended film references cited above. Typically a polymeric film such as a polyethylene (LDPE, LLDPE, MDPE, HOPE or laminates thereof) or preferably a monolithic polymeric film is heated close to its melting point and exposed through a forming screen to a suction force which pulls those areas exposed to the force into the forming apertures which are shaped such that the film is formed into that shape and, when the suction force is high enough, the film breaks at its end thereby forming an aperture through the film.

Especially using a monolithic polymer film as the material for the first layer provides water vapor permeability even under stress conditions. While the apertures provide air permeability during "leakage safe" situations but close the capillaries under stress conditions the monolithic material maintains water vapor permeability in such a case. Preferred breathable monolithic film materials for use herein are those having a high vapor exchange. Suitable monolithic films include Hytrel (TM), available from DuPont Corporation, USA, and other such materials as described in Index 93 Congress, Session 7A "Adding value to Nonwovens", J-C. Cardinal and Y. Trouilhet, DuPont de Nemours international S.A, Switzerland.

Various forms, shapes, sizes and configurations of the capillaries are disclosed in EP-A-934735 and EP-A-934736 both of which are herein incorporated for reference. In particular the apertures form capillaries which have side walls. The capillaries extend away from the wearer facing surface of the film for a length which typically should be at least in the order of magnitude of the largest diameter of the aperture while this distance can reach up to several times the largest aperture diameter. The capillaries have a first opening in the plane of the garment facing surface of the film and a second opening which is the opening formed when the suction force (such as a vacuum) in the above mentioned process creates the aperture. Naturally the edge of the second opening may be rugged or uneven, comprising loose elements extending from the edge of the opening. However, it is preferred that the opening be as smooth as possible so as not to create a liquid transport entanglement between the extending elements at the end of the second opening of the capillary with the absorbent core in the absorbent article (in contrast this may be desirable for apertured film topsheets where such loose elements provide the function of sucker feet). The capillaries in the first layer of the breathable backsheet allow air and water vapor permeability which is not hindered by them being slanted at an angle or by the shape. At the same time the slanting and shaping will allow the capillaries to close under pressure excerpted from the wearer facing side on them such that liquid transport through the capillaries towards the outside of the article becomes nearly impossible. Hence these three-dimensional formed film layers are highly preferable in the context of breathable absorbent articles and in particular so with the additional second outer layer which is provided as hereinafter explained.

The second outer layer of the breathable backsheet according to the present invention is a fibrous nonwoven web having a basis weight of less than 40 g/m2, preferably of less than 28 g/m2. More preferably, the second outer layer is a fibrous nonwoven web formed by a layered composite of a meltblown nonwoven layer made from synthetic fibers having a basis weight of less than 13 g/m2 and of a spunbonded nonwoven layer also made from synthetic fibers.

In the most preferred embodiment herein the backsheet comprises at least a first layer of a resilient, three dimensional web which consists of a liquid impervious polymeric film having apertures forming capillaries which are not perpendicular to the plane of the film but are disposed at an angle of less than 90° relative to the plane of the film, and at least a second breathable layer of a porous web which is a fibrous nonwoven composite web of a meltblown nonwoven layer made from synthetic fibers having a basis weight of less than 13 g/m2 and of a spunbonded nonwoven layer made from synthetic fibers.

Using as the breathable backsheet in the absorbent article of the present invention, a backsheet comprising at least one breathable layer of a resilient, three dimensional web which consists of a liquid impervious polymeric film having apertures forming capillaries which are not perpendicular to the plane of the film but are disposed at an angle of less than 90° relative to the plane of the film, and at least another breathable layer of a porous web which consists of a fibrous nonwoven web having a basis weight of less than 40 g/m2 (particularly of about 28 g/m2), further contributes to the outstanding benefit of the present invention. Indeed these backsheet functions very well in term of comfort, soiling of the user panty, dryness, etc. while providing additional comfort due to the reduced basis weight of the non-woven layer. This reduction of basis weight also provides an improved material consumption structure of the whole article.

### Absorbent core

According to the present invention the absorbent articles may further comprise a topsheet and absorbent core. The absorbent material or core can be a fluffy fibrous absorbent core, comprising hydrogel particles if desired, or laminated tissues with or without particulate materials including hydrogel particles. The absorbent core fibres can be any of those known in the art including cellulose fibres or polymeric fibres rendered absorbent or even non absorbent matrix fibres. Also tissues of sufficient basis weight and absorbency can be used in the absorbent core according to the present invention.

According to the present invention, the absorbent can include the following components: (a) an optional primary fluid distribution layer preferably together with a secondary optional fluid distribution layer; (b) a fluid storage layer; (c) an optional fibrous ("dusting") layer underlying the storage layer; and (d) other optional components. According to the present invention the absorbent may have any thickness depending on the end use envisioned.

### a Primary/Secondary Fluid Distribution Layer

One optional component of the absorbent 'according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers. In particular fibrous layers maintain the capillaries between fibers even when wet are useful as distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials in combination with suitable carriers.

Suitable carriers include materials which are conventionally utilized in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention may comprise multiple layers comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

### Topsheet

According to the present invention the topsheet may comprise a single layer or a multiplicity of layers. In a preferred embodiment the topsheet comprises a first layer which provides the user facing surface of the topsheet and a second layer between the first layer and the absorbent structure/core. The topsheet provides a layer through which the liquids to be absorbed penetrate to the absorbent material.

The topsheet as a whole and hence each layer individually needs to be compliant, soft feeling, and non-irritating to the wearer's skin. It also can have elastic characteristics allowing it to be stretched in one or two directions. Typically, the topsheet extends across the whole of the absorbent structure and can extend into and form part of or all of the preferred sideflaps, side wrapping elements or wings. According to the present invention the topsheet may be formed from any of the materials available for this purpose and known in the art, such as woven non woven materials, polymeric materials such as apertured formed thermoplastic films, apertured plastic films and hydroformed thermoplastic films, thermoplastic scrims or combinations thereof. Suitable woven and non woven materials can be comprised of natural fibers (e.g., wood or cotton fibers), synthetic fibers (e.g., polymeric fibers such as polyester, polypropylene or polyethylene fibers) or from a combination of natural and synthetic fibers or bi-/multi- component fibers and are preferably hydrophobic.

In a preferred embodiment of the present invention at least one of the layers of the topsheet comprises a hydrophobic, liquid permeable apertured polymeric film. Preferably, the upper layer is provided by a film material having apertures which are provided to facilitate liquid transport from the wearer facing surface towards the absorbent structure, as detailed for example in US 3 929 135, US 4 151 240, US 4 319 868, US 4 324 426, US 4 343 314, US 4 463 045, US 5 006 394 and US 4 591 523. Apertured formed films are especially preferred for the topsheets because they are pervious to body exudates and yet non absorbent and have a reduced tendency to allow fluids to pass back through and rewet the wearer's skin. Thus, the surface of the formed film that is in contact with the body remains dry, thereby reducing body soiling and creating a more comfortable feel for the wearer. Particularly preferred micro apertured formed film topsheets are disclosed in US 4 609 518 and US 4 629 643.

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The wearer facing surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in WO 93/09741. Alternatively, the wearer facing surface of the topsheet can be made hydrophilic by treating it with a surfactant such as is described in US 4 950 254.

According to the present invention the absorbent article is constructed by joining the various elements such as topsheet, backsheet and absorbent core by any means well known in the art. For example the backsheet and/ or topsheet may be joined to the absorbent core or to each other by a uniform continuous layer of adhesive, a patterned layer of adhesive, or an array of separate lines, spirals or spots of adhesive. Alternatively, the elements may be joined by heat bonds, pressure bonds, ultra sonic bonds, dynamic mechanical bonds or any other suitable joining means known in the art and any combination thereof. Preferably the breathable backsheet is bonded to other elements of the absorbent article so as to minimise and preferably eliminate any reduction in the vapour permeability of the backsheet.

According to the present invention the absorbent article may find utility as sanitary napkins, panty liners, adult incontinence products and baby diapers. The present invention finds particular susceptibility as sanitary napkins and panty liners. Thus in addition to the components described herein above, the absorbent article may also comprise all those features and parts which are typical for products in the context of their intended use such as wings and side flaps, undergarment adhesive means, release paper, wrapping elements, fastening means and the like.

### Odor control test

The odor reduction is measured by for example an in vitro sniff test. In vitro sniff test consists in analyzing by expert graders the odor associated with articles comprising the ingredients to be tested (including references articles) when contacted with an odourous components-containing solution.

The expert graders express their judgment about (un)pleasantness of the odor using a (un)pleasantness scale, typically from -10 (highest level of unpleasantness) to 5 (most pleasant). With this procedure, each grader compares MU (Unpleasantness) in the test session. The relative MU odor values from different products are assigned numbers. For example, in a test session, a sample that is perceived to be twice as strong as another is assigned twice as large a number. One that is perceived to be one-tenth as strong as another is assigned a number one-tenth as large, etc. In each test session, zero is used to designate neutral hedonicity, and + and - numbers are assigned in ratio proportion to the relative pleasantness and unpleasantness of the odor.

Surprisingly in vitro in-house sniff tests conducted by using an odourous components-containing solution reproducing the essential malodorous characteristics of menses showed synergistic odor reduction when comparing lactic acid producing microorganisms (e.g. B coagulans) together with odour absorbing agent (e.g., silicate available as Syloblanc 82 from Grace GmbH and/or zeolite available as Zeolite A, Wessalith CSfrom Degussa AG) to each of these ingredients taken alone at the same total level of active. Indeed the % of unpleasantness reduction obtained for the mixture was higher than the % of unpleasantness reduction obtained for each of the two ingredients used alone at the same total level of active. The Unpleasantness values, for each sample, was obtained as a mean of at least 15 observations ( 3 products, 5 graders). These results were statistically significant.

Alternatively the odor reduction can also be measured with in vivo sniff tests as described in patent applications, EP-A-811387 or WO97/46191, herein incorporated by reference.

The present invention is further illustrated by the following examples

### Examples

### Example 1:

This is an example of a panty liner according to the present invention and is a modified panty liner based on Always "Alldays Duo Active" manufactured by Procter & Gamble, Germany. The topsheet is a film/non woven composite {film supplier code BPC 5105 CPM BP Chemical Germany, non woven supplier code ARBO TB/BI Mequinenza Spain}. The core material is a tissue laminate (13.2 cm x 4.0 cm) composed of a 2 layers of airlayed tissue of 55 g/m² basis weight {available from Unikay Italy under the supplier code Unikay 303 LF}. Between the two tissue layers the laminate contains B coagulans commercially available from ATCC under number 31284 in a freeze-dried form, such that the pantyliner contains about 10⁹ cfu of such microorganisms.

The backsheet comprises two layers a first layer and a second layer. The first layer is in contact with the absorbent tissue and the second layer. The second layer is in contact with the first layer and the undergarment of the wearer. The first layer is a formed apertured film (CPT) made of Low Density PE {supplied by Tredegar Film Products B.V. Holland under the manufacturing code X-1522}. The second layer is composed of a nonwoven laminate {13MB/16SB manufactured by Corovin GmbH in Germany under the trade name MD 2005}. The nonwoven laminate is composed of 16 g/m² spunbond and 13 g/m² meltblown. Each backsheet layer is joined over the full surface by a extensively overlapped spiral glue application at a basis weight of approximately 8 g/m². The glue utilised for attachment of both backsheet layers was supplied by SAVARE' SpA. Italy (under the material code PM17).

### Example 2

Example 2 is identical to example 1 except that the second layer of the backsheet has been replaced by a nonwoven laminate composed of 16g/m² spunbond and 6 g/m² meltblown {supplied under the code of SM 22-6PH by Union SpA, Italy}.

### Example 3

Example 3 is identical to example 1 except that AGM was added (cross-linked sodium polyacrylate XZ 9589001 available from Dow Chemicals ) at a basis weight of 60 g/m^{2 .}

### Example 4

Example 4 is identical to example 3 except that the second layer of the backsheet has been replaced by a nonwoven laminate composed of 16g/m² spunbond and 6 g/m² meltblown {supplied under the code of SM 22-6PH by Union SpA, Italy}.

### Example 5

This is an example of a sanitary napkin according to the present invention. The sanitary napkin is based on an Always Ultra sanitary napkin available from Procter & Gamble Germany which has been modified. The topsheet is a CPM material available from Tredegar Film Products B. V. Holland under the code X-1522. The core material is a tissue laminate (20.7 cm x 7.0 cm) composed of a 2 layers of airlayed tissue of 55 g/m² basis weight {available from Unikay Italy under the supplier code Unikay 303 LF}. Between the two tissue layers the laminate contains AGM (available from DOW Chemicals Germany under the supplier code; DOW XZ 95890.1) at a basis weight of 60 g/m², zeolite (available from Degussa Germany under the supplier code; Wessalith CS) at a basis weight of 61 g/m² and B.coagulans commercially available from ATCC under number 31284 in a freeze-dried form, such that the napkin contains about 10⁹ cfu of such microorganisms.

The core laminate was manufactured and supplied by Korma Italy (under the experimental manufacturing code: XA 070.01.003). The sanitary napkin has a multi-layer breathable backsheet comprising a formed apertured film backsheet layer and a second nonwoven layer. The first layer is a blend of low and high density PE with a crush resistant hexagonal hole configuration {supplied by Tredegar Film Products B.V. Holland under the manufacturing code AS 225 HD 25}. The second layer is an improved nonwoven laminate composed of 3 layers with basis weights 14g/m² spunbond - 20 g/m² meltblown - 14 g/m² spunbond (manufactured by Corovin GmbH in Germany under the trade name MD 3005).

### Example 6

Example 6 is identical to example 5 except that the second layer of backsheet has been replaced by a microporous layer ( manufactured by Exxon Chemical Company in Illinois under the name Exxon XBF 112W) composed of Low Density PE and calcium carbonate particles at basis weight of 35 g/m2.

### Example 7

Example 7 is identical to example 5 except that the first layer of backsheet has been replaced by an improved resilient tri-dimensional web (supplied by Tredegar Film Products B.V. Holland under the manufacturing code V174LD40) which consist of a blend of low density PE having apertures forming capillaries which are not perpendicular to the plane of the film but are disposed at an angle of less than 90° relative to the plane of the film and that the second layer of the backsheet has been replaced by a nonwoven laminate manufactured by Corovin GmbH (BBA Group) in Germany under the manufacturing code V 8/6. The nonwoven laminate is composed of 16 g/m² basis weight spunbond fiber layer and 11.5 g/m² basis weight meltblown fiber layer (thus a total basis weight of 27.5 g/m2).

### Example 8

This is an example of a pantyliner according to the present invention and is a modified panty liner based on Always "Alldays Duo Active" manufactured by Procter & Gamble, Germany. The topsheet is a film/non woven composite {film supplier code BPC 5105 CPM BP Chemical Germany, non woven supplier code ARBO TB/BI Mequinenza Spain}. The core material is a tissue laminate (13.2 cm x 4.0 cm) composed of a 2 layers of airlayed tissue of 55 g/m² basis weight {available from Unikay Italy under the supplier code Unikay 303 LF}. Between the two tissue layers the laminate contains B. coagulans spores Lactospore® commercially available from Sabinsa Corporation (Sochim International S.P.A. Milano) at a basis weight of 55 g/m^{2.} This corresponds to about 10*10⁹ spores (ten billions of spores per pantiliner). 10*10⁹ spores correspond to 10*10⁹ cfu of B. coagulans per pantiliner.

The backsheet comprises two layers a first layer and a second layer. The first layer is in contact with the absorbent tissue and the second layer. The second layer is in contact with the first layer and the undergarment of the wearer. The first layer is a formed apertured film (CPT) made of Low Density PE {supplied by Tredegar Film Products B.V. Holland under the manufacturing code X-1522}. The second layer is composed of a nonwoven laminate {13MB/16SB manufactured by Corovin GmbH in Germany under the trade name MD 2005}. The nonwoven laminate is composed of 16 g/m² spunbond and 13 g/m² meltblown. Each backsheet layer is joined over the full surface by a extensively overlapped spiral glue application at a basis weight of approximately 8 g/m². The glue utilised for attachment of both backsheet layers was supplied by SAVARE' SpA. Italy (under the material code PM17).

### Example 9

Example 9 is identical to example 8 except that the second layer of the backsheet has been replaced by a nonwoven laminate composed of 16g/m² spunbond and 6 g/m² meltblown {supplied under the code of SM 22-6PH by Union SpA, Italy}.

### Example 10

Example 10 is identical to example 8 except that AGM was added (cross-linked sodium polyacrylate XZ 9589001 available from Dow Chemicals) at a basis weight of 60 g/m².

### Example 11

Example 11 is identical to example 10 except that the second layer of the backsheet has been replaced by a nonwoven laminate composed of 16g/m² spunbond and 6 g/m² meltblown {supplied under the code of SM 22-6PH by Union SpA, Italy}.

### Example 12

This is an example of a sanitary napkin according to the present invention. The sanitary napkin is based on an Always Ultra sanitary napkin available from Procter & Gamble Germany which has been modified. The topsheet is a CPM material available from Tredegar Film Products B. V. Holland under the code X-1522. The core material is a tissue laminate (20.7 cm x 7.0 cm) composed of a 2 layers of airlayed tissue of 55 g/m² basis weight {available from Unikay Italy under the supplier code Unikay 303 LF}. Between the two tissue layers the laminate contains an odor control system of AGM (available from DOW Chemicals Germany under the supplier code; DOW XZ 95890.1) at a basis weight of 60 g/m², a zeolite (available from Degussa Germany under the supplier code; Wessalith CS) at a basis weight of 61 g/m² and B. coagulans spores Lactospore® commercially available from Sabinsa Corporation (Sochim International S.P.A. Milano) at a basis weight of 55 g/m². This corresponds to about 10*10⁹ spores (ten billions of spores per napkin). 10*10⁹ spores correspond to 10*10⁹ cfu of B. coagulans per napkin.

The core laminate was manufactured and supplied by Korma Italy (under the experimental manufacturing code: XA 070.01.003). The sanitary napkin has a multi-layer breathable backsheet comprising a formed apertured film backsheet layer and a second nonwoven layer. The first layer is a blend of low and high density PE with a crush resistant hexagonal hole configuration {supplied by Tredegar Film Products B.V. Holland under the manufacturing code AS 225 HD 25}. The second layer is an improved nonwoven laminate composed of 3 layers with basis weights 14g/m² spunbond - 20 g/m² meltblown - 14 g/m² spunbond (manufactured by Corovin GmbH in Germany under the trade name MD 3005).

### Example 13

Example 13 is identical to example 12 except that the second layer of backsheet has been replaced by a microporous layer (manufactured by Exxon Chemical Company in Illinois under the name Exxon XBF 112W) composed of Low Density PE and calcium carbonate particles at basis weight of 35 g/m2.

### Example 14

Example 14 is identical to example 12 except that the first layer of backsheet has been replaced by an improved resilient tri-dimensional web (supplied by Tredegar Film Products B.V. Holland under the manufacturing code V174LD40) which consist of a blend of low density PE having apertures forming capillaries which are not perpendicular to the plane of the film but are disposed at an angle of less than 90° relative to the plane of the film and that the second layer of the backsheet has been replaced by a nonwoven laminate manufactured by Corovin GmbH (BBA Group) in Germany under the manufacturing code V 8/6. The nonwoven laminate is composed of 16 g/m² basis weight spunbond fiber layer and 11.5 g/m² basis weight meltblown fiber layer (thus a total basis weight of 27.5 g/m2).

### Example A

The feminine pads used in the following examples were Always (Always is a registered Trade Mark) as sold by the Procter & Gamble Company.

Each feminine pad was opened by cutting the wrap around the perforated coverstock at its bottom face approximately along a longitudinal edge of the release paper which covers the external adhesive layer. The side of the absorbent fibrous core was then exposed by slightly shifting the water impermeable plastic bottom layer and subsequently, the fibrous core was split into two halves, each having approximately the same thickness, along a plane which is parallel to the plane of the napkin itself. The odor adsorbing agents (Silica or Zeolite or both) were homogeneously mixed together with lactic aid producing microorganisms and homogeneously distributed on this layer. Then all the layers were joined together to reconstitute the absorbent core.

The water impermeable inner backsheet was then put back into its original position and the wrap around perforated coverstock was sealed along the cut by means of e.g. a double sided adhesive tape.

Samples were produced using the method above, containing the odor control systems as described hereinbelow.

The lactic acid producing microorganisms used were B. coagulans spores powder commercially available as Lactospore® (0.7g) from Sabinsa Corporation (Sochim International S.P.A. Milano). Thus each pad comprised about 10*10⁹cfu of L.sporogenes. The silica (1g) used was Syloblanc 82 available from Grace GmbH. The zeolite (0.8g) used was Zeolite A, Wessalith CS, available from Degussa AG.

Alternatively other samples were prepared with B. coagulans (living form) commercially available under ATCC number 31284 in freeze-dried form instead of Lactospore®, the samples comprising about 10⁹cfu of such microorganisms.

### Example B

In Example B samples were produced using the same method as in Example A, except that AGM (0.8g) cross-linked sodium polyacrylate XZ 9589001 available from Dow Chemicals, was added to the lactic acid producing microorganisms and odor absorbing agents (silicate or zeolite or both).

### Example C

Other pads were prepared by following the method in example A except that after having split the fibrous core into two halves, the lactic acid producing microorganisms powder was homogeneously distributed onto the upper halve fibrous layer (i.e. the fibrous layer halve intended to be closer to the topsheet) and the odour absorbing agent (Silicate and/or Zeolite) was homogeneously distributed onto the lower halve fibrous layer (i.e., the one intended to be closer to the backsheet of the pad once reconstituted). Then a layer of airlaid tissue (19 mm* 70 mm of low basis weight) (available from Fripa under the code/name NCB Tissue HWS) was positioned between the two halve fibrous layers which are then joined together to reconstitute the absorbent core. The presence of the airlaid tissue between the two fibrous layer avoids direct contact between the lactic acid producing microorganisms and the odor absorbing agents.

These samples were produced using as the lactic acid producing microorganisms powder, B. coagulans spores powder commercially available as Lactospore® (0.7g) from Sabinsa Corporation (Sochim International S.P.A. Milano). Thus the pads comprised about 10 * 10⁹ cfu of B. coagulans. The silica (0.8g) used was Syloblanc 82 available from Grace GmbH. The zeolite (0.8g) used was Zeolite A, Wessalith CS, available from Degussa AG.

Alternatively other samples were prepared with B. coagulans (living form) commercially available under ATCC number 31284 in freeze-dried form instead of Lactospore®, the samples comprising about 10⁹cfu of such microorganisms.

### Example D

In Example D samples were produced using the same method as in Example C, except that AGM (0.8g) cross-linked sodium polyacrylate XZ 9589001 available from Dow Chemicals, was added to the odor absorbing agents (silicate or zeolite or both) onto the lower halve fibrous layer.

All the pads illustrated in examples A to D were found to provide outstanding odour control benefits when in contact with bodily fluids. Indeed a synergistic odour reduction was observed when comparing these samples to samples containing only odour absorbing agents (zeolite and/or silicate) or only the microorganisms herein at same total level of total odour control materials.

## Claims

1. An absorbent article comprising a liquid permeable topsheet, a breathable backsheet and an absorbent core, said core being intermediate said topsheet and said backsheet, said absorbent article comprising lactic acid producing microorganisms.

2. An absorbent article according to claim 1, wherein said lactic acid producing microorganisms are selected from the genera Lactobacillus, Lactococcus, Pedioccocus, Leuconostoc, Sporolactobacillus, Bacillus or a mixture thereof, and preferably from the species Bacillus coagulans, Bacillus subtilis, Bacillus laterosporus, Bacillus laevolacticus, Sporolactobacillus inulinus, Lactobacillus acidophilus, Lactobacillus curvatus, Lactobacillus plantarum, Lactobacillus jenseni, Lactobacillus casei, Lactobacillus fermentum, Lactococcus lactis, Pedioccocus acidilacti, Pedioccocus pentosaceus, Pedioccocus urinae, Leuconostoc mesenteroides or mixtures thereof.

3. An article according to any of the preceding claims wherein the lactic acid producing microorganisms are spore-forming lactic acid producing microorganisms and preferably is the species B. coagulans in its living form or dormant form (spore).

4. An article according to any of the preceding claims wherein the absorbent article comprises more than 10⁴ cfu, preferably more than 10⁶ cfu, more preferably from 10⁷ to 10¹² cfu of lactic acid producing microorganisms.

5. An article according to any of the preceding claims, which further comprises an absorbent gelling material or a mixture thereof, preferably a partially neutralized absorbent gelling material.

6. An article according to claim 5, wherein the level of the absorbent gelling material or a mixture thereof is from 0 gm⁻² to 300gm⁻², preferably from 30gm⁻² to 110gm⁻², more preferably from 55gm⁻² to 85gm⁻².

7. An absorbent article according to any one of the preceding claims, which further comprises at least an additional odor control agent and preferably an odour absorbing agent or mixtures thereof.

8. An absorbent article according to claim 7, wherein said additional odor control agent is selected from the group consisting of silicas, zeolites, carbons, starches, cyclodextrine, kieselguhr, clays, ion exchange resins, carbonates, bicarbonates, phosphates, sulphates, masking agents and combination thereof and preferably is silicate, zeolite or a combination thereof.

9. An article according to any of the preceding claims 7 to 8 which comprises from 0 gm⁻² to 600 gm⁻², preferably from 5 to 500 gm⁻², and most preferably from 20 gm⁻² to 200 gm⁻² of said additional odor control agent or a mixture thereof.

10. An absorbent article according to any one of the preceding claims, wherein said breathable backsheet comprises at least one layer selected from an apertured polymeric film or a 2-dimensional planar apertured film.

11. An absorbent article according to claim 10, wherein said layer is a 2 dimensional planar apertured layer, wherein said apertures have an average diameter of from 150 micrometers to 5 micrometers.

12. An absorbent article according to claim 10, wherein said layer is an apertured polymeric film, wherein said apertures have an average diameter of from 100 micrometers to 500 micrometers.

13. An absorbent article according to claim 10, wherein said breathable backsheet comprises at least two layers, a first layer comprising an apertured layer and a second layer comprising a fibrous layer.

14. An absorbent article according to claim 13, wherein said breathable backsheet comprises at least a first layer of a resilient, three dimensional web which consists of a liquid impervious polymeric film having apertures forming capillaries which are not perpendicular to the plane of the film but are disposed at an angle of less than 90° relative to the plane of the film, and at least a second breathable layer being a fibrous nonwoven web made from synthetic fibers having a basis weight of less than 40 g/m2.

15. An absorbent article according to any one of the preceding claims, wherein said article is a sanitary napkin, a nursing pad, baby diaper or a panty liner.

16. The use of lactic acid producing microorganisms in a breathable absorbent article, comprising a liquid permeable topsheet, an absorbent core and a breathable backsheet, for reducing leakage/wet through.

17. The use of lactic acid producing microorganisms in a breathable absorbent article, comprising a wearing facing surface and a garment facing surface for improved dryness of the wearing facing surface.

18. The use according to claim 17 wherein said absorbent article further comprises a topsheet, a backsheet and an absorbent core, said core being located in between said topsheet and said backsheet and wherein said topsheet provides said wearing facing surface.

19. A disposable absorbent article, for controlling odours, preferably odours associated with bodily fluids, comprising lactic acid producing micro-organisms and at least one odour absorbing agent selected from the group consisting of silicas, zeolites, carbons, starches, cyclodextrine, kieselguhr, clays, ion exchange resins and combination thereof and preferably is a silicate, a zeolite or a combination thereof and is present at a level of from 20 to 600 gm⁻², more preferably from 40 to 500 gm⁻², most preferably from 100 to 400 gm⁻² per article.

20. An article according to claim 19, wherein said lactic acid producing microorganisms are selected from the genera Lactobacillus, Lactococcus, Pedioccocus, Leuconostoc, Sporolactobacillus, Bacillus or a mixture thereof and preferably from the species Bacillus coagulans, Bacillus subtilis, Bacillus laterosporus, Bacillus laevolacticus, Sporolactobacillus inulinus, Lactobacillus acidophilus, Lactobacillus curvatus, Lactobacillus plantarum, Lactobacillus jenseni, Lactobacillus casei, Lactobacillus fermentum, Lactococcus lactis, Pedioccocus acidilacti, Pedioccocus pentosaceus, Pedioccocus urinae, Leuconostoc mesenteroides or mixture thereof.

21. An article according to any of the preceding claims 19 or 20 wherein the lactic acid producing microorganisms are spore forming lactic acid producing microorganisms and preferably is the species B. coagulans in its living form or dormant form (spore).

22. An article according to any of the preceding claims 19 to 21 wherein the article comprises more than 10² cfu, preferably more than 10⁴ cfu and more preferably from 10⁷ to 10¹⁰ cfu of said lactic acid producing microorganisms.

23. An article according to any of the preceding claims 19 to 22 which comprises spores of lactic acid producing microorganisms, preferably of the species Bacillus coagulants together with both silica and zeolite as the odor absorbing agent.

24. An article according to any of the preceding claims 19 to 23 which further comprises an absorbent gelling material, typically at a level of from 10gm⁻² to 300gm⁻², preferably from 30gm⁻² to 150gm⁻², more preferably from 55gm⁻² to 85gm⁻² per article.

25. An article according to any of the preceding claims 19 to 24 wherein said article is a disposable absorbent article, preferably a sanitary napkin, pantiliner, tampon, diaper, incontinent pad, breast pad, perspiration pad, human or animal waste management device or interlabial pad.

26. An article according to any of the preceding claims 19 to 25 wherein said article is an absorbent disposable article comprising a liquid pervious topsheet, a backsheet and an absorbent core intermediate said backsheet and said topsheet.

27. The use, in a disposable absorbent article, as an odor control means, of lactic acid producing microorganisms, preferably spore-forming lactic acid-producing microorganisms and more preferably the species Bacillus coagulants, together with an odor absorbing agent, said agent being selected from the group consisting of silicas, zeolites, carbons, starches, cyclodextrine, kieselguhr, clays, ion exchange resins and combination thereof and preferably is a silicate, a zeolite or a combination thereof and is present at a level of from 20 to 600 gm⁻², more preferably from 40 to 500 gm⁻², most preferably from 100 to 400 gm⁻² per article.

## Patentansprüche

1. Absorptionsartikel, umfassend eine flüssigkeitsdurchlässige Oberschicht, eine atmungsaktive untere Schicht und einen Absorptionskern, wobei der Kern zwischen der Oberschicht und der unteren Schicht liegt und wobei der Absorptionsartikel Milchsäure produzierende Mikroorganismen umfasst.

2. Absorptionsartikel nach Anspruch 1, wobei die Milchsäure produzierenden Mikroorganismen ausgewählt sind aus den Gattungen Lactobacillus, Lactococcus, Pedioccocus, Leuconostoc, Sporolactobacillus, Bacillus oder einer Mischung davon und vorzugsweise aus den Arten Bacillus coagulans, Bacillus subtilis, Bacillus laterosporus, Bacillus laevolacticus, Sporolactobacillus inulinus, Lactobacillus acidophilus, Lactobacillus curvatus, Lactobacillus plantarum, Lactobacillus jenseni, Lactobacillus casei, Lactobacillus fermentum, Lactococcus lactis, Pedioccocus acidilacti, Pedioccocus pentosaceus, Pedioccocus urinae, Leuconostoc mesenteroides oder Mischungen davon.

3. Artikel nach einem der vorstehenden Ansprüche, wobei die Milchsäure produzierenden Mikroorganismen sporenbildende Milchsäure produzierende Mikroorganismen sind und vorzugsweise die Art B. coagulans in ihrer lebenden Form oder ruhenden Form (Spore).

4. Artikel nach einem der vorstehenden Ansprüche, wobei der Absorptionsartikel mehr als 10⁴ cfu, vorzugsweise mehr als 10⁶ cfu, bevorzugter 10⁷ bis 10¹² cfu Milchsäure produzierende Mikroorganismen umfasst.

5. Artikel nach einem der vorstehenden Ansprüche, der weiterhin ein Absorptionsgeliermaterial oder eine Mischung davon, vorzugsweise ein teilweise neutralisiertes Absorptionsgeliermaterial umfasst.

6. Artikel nach Anspruch 5, wobei die Konzentration des Absorptionsgeliermaterials oder einer Mischung davon 0 gm⁻¹ bis 300 gm⁻², vorzugsweise 30 gm⁻² bis 110 gm⁻², bevorzugter 55 gm⁻² bis 85 gm⁻² beträgt.

7. Absorptionsartikel nach einem der vorstehenden Ansprüche, der weiterhin mindestens ein zusätzliches Geruchsbekämpfungsmittel und vorzugsweise ein geruchsabsorbierendes Mittel oder Mischungen davon umfasst.

8. Absorptionsartikel nach Anspruch 7, wobei das zusätzliche Geruchsbekämpfungsmittel ausgewählt ist aus der Gruppe bestehend aus Silicas, Zeolithen, Kohlenstoffen, Stärken, Cyclodextrinen, Kieselgur, Tonerden, Ionenaustauscherharzen, Carbonaten, Bicarbonaten, Phosphaten, Sulfaten, Maskierungsmitteln und Kombinationen davon und vorzugsweise Silikat, Zeolith oder eine Kombination davon ist.

9. Artikel nach einem der vorstehenden Ansprüche 7 bis 8, der 0 gm⁻² bis 600 gm⁻², vorzugsweise 5 bis 500 gm⁻² und am meisten bevorzugt 20 gm⁻² bis 200 gm⁻² zusätzliches Geruchsbekämpfungsmittel oder eine Mischung davon umfasst.

10. Absorptionsartikel nach einem der vorstehenden Ansprüche, wobei die atmungsaktive untere Schicht mindestens eine Lage umfasst, die aus einer perforierten Polymerfolie oder einer zweidimensionalen planaren perforierten Folie ausgewählt ist.

11. Absorptionsartikel nach Anspruch 10, wobei die Lage eine zweidimensionale planare perforierte Lage ist, wobei die Perforierungen einen durchschnittlichen Durchmesser von 150 Mikrometer bis 5 Mikrometer aufweisen.

12. Absorptionsartikel nach Anspruch 10, wobei die Lage eine perforierte Polymerfolie ist, wobei die Perforierungen einen durchschnittlichen Durchmesser von 100 Mikrometer bis 500 Mikrometer aufweisen.

13. Absorptionsartikel nach Anspruch 10, wobei die atmungsaktive untere Schicht mindestens zwei Lagen umfasst, nämlich eine erste Lage, die eine perforierte Lage umfasst, und eine zweite Lage, die eine aus Fasern bestehende Lage umfasst.

14. Absorptionsartikel nach Anspruch 13, wobei die atmungsaktive untere Schicht mindestens eine erste Lage aus einer elastischen, dreidimensionalen Bahn, die aus einer flüssigkeitsundurchlässigen Polymerfolie mit Perforierungen besteht, die Kapillaren bilden, welche nicht senkrecht zur Fläche der Folie verlaufen, sondern in einem Winkel von weniger als 90° zur Fläche der Folie angeordnet sind, und mindestens eine zweite atmungsaktive Lage, die eine Faservliesbahn aus synthetischen Fasern mit einem Grundgewicht von weniger als 40 g/m² ist, umfasst.

15. Absorptionsartikel nach einem der vorstehenden Ansprüche, wobei der Artikel eine Damenbinde, eine Stilleinlage, eine Babywindel oder eine Slipeinlage ist.

16. Verwendung von Milchsäure produzierenden Mikroorganismen in einem atmungsaktiven Absorptionsartikel, der eine flüssigkeitsdurchlässige Oberschicht, einen Absorptionskern und eine atmungsaktive untere Schicht umfasst, zur Reduzierung von Leckage/Durchnässen.

17. Verwendung von Milchsäure produzierenden Mikroorganismen in einem atmungsaktiven Absorptionsartikel, der eine zum Benutzer gerichtete Oberfläche und eine zum Kleidungsstück gerichtete Oberfläche umfasst, für eine verbesserte Trockenheit der zum Benutzer gerichteten Oberfläche.

18. Verwendung nach Anspruch 17, wobei der Absorptionsartikel weiterhin eine Oberschicht, eine untere Schicht und einen Absorptionskern umfasst, wobei sich der Kern zwischen der Oberschicht und der unteren Schicht befindet und wobei die Oberschicht die zum Benutzer gerichtete Oberfläche bereitstellt.

19. Einwegabsorptionsartikel zum Bekämpfen von Gerüchen, vorzugsweise von mit Körperflüssigkeiten assoziierten Gerüchen, der Milchsäure produzierende Mikroorganismen und mindestens ein geruchsabsorbierendes Mittel umfasst, das ausgewählt ist aus der Gruppe bestehend aus Silicas, Zeolithen, Kohlenstoffen, Stärken, Cyclodextrinen, Kieselgur, Tonerden, Ionenaustauscherharzen, Carbonaten, Bicarbonaten, Phosphaten, Sulfaten, Maskierungsmitteln und Kombinationen davon und vorzugsweise ein Silica, ein Zeolith oder eine Kombination davon ist und in einer Konzentration von 20 bis 600 gm⁻² vorliegt, bevorzugter von 40 bis 500 gm⁻², am meisten bevorzugt von 100 bis 400 gm⁻² pro Artikel.

20. Artikel nach Anspruch 19, wobei die Milchsäure produzierenden Mikroorganismen ausgewählt sind aus den Gattungen Lactobacillus, Lactococcus, Pedioccocus, Leuconostoc, Sporolactobacillus, Bacillus oder einer Mischung davon und vorzugsweise aus den Arten Bacillus coagulans, Bacillus subtilis, Bacillus laterosporus, Bacillus laevolacticus, Sporolactobacillus inulinus, Lactobacillus acidophilus, Lactobacillus curvatus, Lactobacillus plantarum, Lactobacillus jenseni, Lactobacillus casei, Lactobacillus fermentum, Lactococcus lactis, Pedioccocus acidilacti, Pedioccocus pentosaceus, Pedioccocus urinae, Leuconostoc mesenteroides oder Mischungen davon.

21. Artikel nach einem der vorstehenden Ansprüche 19 oder 20, wobei die Milchsäure produzierenden Mikroorganismen sporenbildende Milchsäure produzierende Mikroorganismen sind und vorzugsweise die Art B. coagulans in ihrer lebenden Form oder ruhenden Form (Spore).

22. Artikel nach einem der vorstehenden Ansprüche 19 bis 21, wobei der Artikel mehr als 10² cfu, vorzugsweise mehr als 10⁴ cfu und bevorzugter 10⁷ bis 10¹⁰ cfu der Milchsäure produzierenden Mikroorganismen umfasst.

23. Artikel nach einem der vorstehenden Ansprüche 19 bis 22, der Sporen von Milchsäure produzierenden Mikroorganismen, vorzugsweise der Art Bacillus coagulans zusammen mit sowohl Silica als auch Zeolith als das geruchabsorbierende Mittel umfasst.

24. Artikel nach einem der vorstehenden Ansprüche 19 bis 23, der weiterhin ein Absorptionsgeliermaterial, in der Regel in einer Konzentration von 10 gm⁻² bis 300 gm⁻², vorzugsweise von 30 gm⁻² bis 150 gm⁻², bevorzugter von 55 gm⁻² bis 85 gm⁻² pro Artikel, umfasst.

25. Artikel nach einem der vorstehenden Ansprüche 19 bis 24, wobei der Artikel ein Einwegabsorptionsartikel, vorzugsweise eine Damenbinde, eine Slipeinlage, ein Tampon, eine Windel, eine Inkontinenz-Slipeinlage, eine Stilleinlage, eine Schweißeinlage, eine Vorrichtung zur Handhabung von menschlichen oder tierischen Abfallprodukten oder eine Interlabialeinlage ist.

26. Artikel nach einem der vorstehenden Ansprüche 19 bis 25, wobei der Artikel ein Einwegabsorptionsartikel ist, der eine flüssigkeitsdurchlässige Oberschicht, eine untere Schicht und einen Absorptionskern zwischen der unteren Schicht und der Oberschicht umfasst.

27. Verwendung von milchsäureproduzierenden Mikroorganismen in einem Einwegabsorptionsartikel als ein Mittel zum Bekämpfen von Gerüchen, vorzugsweise von sporenbildenden Milchsäure produzierenden Mikroorganismen und bevorzugter der Species Bacillus coagulans, zusammen mit einem geruchsabsorbierenden Mittel, wobei das Mittel ausgewählt ist aus der Gruppe bestehend aus Silicas, Zeolithen, Kohlenstoffen, Stärken, Cyclodextrinen, Kieselgur, Tonerden, Ionenaustauscherharzen, Carbonaten, Bicarbonaten, Phosphaten, Sulfaten, Maskierungsmitteln und Kombinationen davon und vorzugsweise ein Silica, ein Zeolith oder eine Kombination davon ist und in einer Konzentration von 20 bis 600 gm⁻² vorliegt, bevorzugter von 40 bis 500 gm⁻², am meisten bevorzugt von 100 bis 400 gm⁻² pro Artikel.

## Revendications

1. Article absorbant comprenant une feuille supérieure liquide perméable, une feuille de fond perméable à l'air et une âme absorbante, ladite âme étant intermédiaire entre ladite feuille supérieure et ladite feuille de fond, ledit article absorbant comprenant des micro-organismes produisant de l'acide lactique.

2. Article absorbant selon la revendication 1, où lesdits micro-organismes produisant de l'acide lactique sont choisis parmi les genres Lactobacillus, Lactococcus, Pedioccocus, Leuconostoc, Sporolactobacillus, Bacillus ou leurs mélanges, et de préférence parmi les espèces Bacillus coagulans, Bacillus subtilis, Bacillus laterosporus, Bacillus laevolacticus, Sporolactobacillus inulinus, Lactobacillus acidophilus, Lactobacillus curvatus, Lactobacillus plantarum, Lactobacillus jenseni, Lactobacillus casei, Lactobacillus fermentum, Lactococcus lactis, Pedioccocus acidilacti, Pedioccocus pentosaceus, Pedioccocus urinae, Leuconostoc mesenteroides ou leurs mélanges.

3. Article selon l'une quelconque des revendications précédentes où les micro-organismes produisant de l'acide lactique sont des micro-organismes produisant de l'acide lactique formant des spores et de préférence sont les espèces B. coagulans sous sa forme vivante ou sa forme dormante (spore).

4. Article selon l'une quelconque des revendications précédentes où l'article absorbant comprend plus de 10⁴ cfu, de préférence plus de 10⁶ cfu, le plus préférablement de 10⁷ à 10¹² cfu de micro-organismes produisant de l'acide lactique.

5. Article selon l'une quelconque des revendications précédentes, qui comprend en outre un matériau absorbant et gélifiant ou ses mélanges, de préférence un matériau absorbant et gélifiant partiellement neutralisé.

6. Article selon la revendication 5, où les teneurs en matériau absorbant et gélifiant ou ses mélanges vont de 0 gm⁻² à 300 gm⁻², de préférence de 30 gm⁻² à 110 gm⁻², le plus préférablement de 55 gm⁻² à 85 gm⁻².

7. Article absorbant selon l'une quelconque des revendications précédentes, qui comprend en outre au moins un agent de contrôle des odeurs supplémentaire, et de préférence un agent absorbant les odeurs ou ses mélanges.

8. Article absorbant selon la revendication 7, où ledit agent de contrôle des odeurs supplémentaire est choisi parmi le groupe constitué de silices, zéolites, carbones, amidons, cyclodextrine, terre à diatomées, argiles, résines échangeuses d'ions, carbonates, bicarbonates, phosphates, sulfates, agents de masquage et leurs combinaisons, et de préférence est un silicate, de la zéolite ou leur combinaison.

9. Article selon l'une quelconque des revendications précédentes 7 à 8 qui comprend de 0 gm⁻² à 600 gm⁻², de préférence de 5 à 500 gm⁻², et le plus préférablement de 20 gm⁻² à 200 gm⁻² dudit agent de contrôle des odeurs supplémentaire ou ses mélanges.

10. Article absorbant selon l'une quelconque des revendications précédentes, où ladite feuille de fond perméable à l'air comprend au moins une couche choisie parmi un film polymère perforé ou un film perforé plan bi-dimensionnel.

11. Article absorbant selon la revendication 10, dans lequel ladite couche est une couche perforée planaire bi-dimensionnelle, dans lequel lesdites perforations ont un diamètre moyen de 150 micromètres à 5 micromètres.

12. Article absorbant selon la revendication 10, dans lequel ladite couche est un film polymère perforé, dans lequel lesdites perforations ont un diamètre moyen de 100 micromètres à 500 micromètres.

13. Article absorbant selon la revendication 10, dans lequel ladite feuille de fond perméable à l'air comprend au moins deux couches, une première couche comprenant une couche perforée et une deuxième couche comprenant une couche fibreuse.

14. Article absorbant selon la revendication 13, où ladite feuille de fond perméable à l'air comprend au moins une première couche d'un voile tridimensionnel résistant qui est constitué d'un film polymère imperméable aux liquides, ayant des perforations formant des capillaires qui ne sont pas perpendiculaires au plan du film mais sont disposées selon un angle de 90 ° par rapport au plan du film, et au moins une seconde couche perméable à l'air étant un voile de nontissé fibreux fait de fibres synthétiques ayant une masse surfacique inférieure à 40 g/m².

15. Article absorbant selon l'une quelconque des revendications précédentes, où ledit article est une serviette hygiénique, une compresse d'allaitement, une couche pour bébés ou un protège-slip.

16. Utilisation de micro-organismes produisant de l'acide lactique dans un article absorbant perméable à l'air, comprenant une feuille de dessus perméable aux liquides, une âme absorbante et une feuille de fond perméable à l'air, pour réduire une fuite/une imprégnation.

17. Utilisation de micro-organismes produisant de l'acide lactique dans un article absorbant perméable à l'air, comprenant une surface faisant face au porteur et une surface faisant face au vêtement pour une meilleure sécheresse de la surface faisant face au porteur.

18. Utilisation selon la revendication 17 dans laquelle ledit article absorbant comprend en outre une feuille de dessus, une feuille de fond et une âme absorbante, ladite âme étant localisée entre ladite feuille de dessus et ladite feuille de fond et où ladite feuille de dessus fournit ladite surface faisant face à l'utilisateur.

19. Article absorbant jetable, pour contrôler les odeurs, de préférence les odeurs associées aux fluides corporels, comprenant des micro-organismes produisant de l'acide lactique et au moins un agent absorbant les odeurs choisi parmi le groupe constitué de silices, zéolites, carbones, amidons, cyclodextrine, terre à diatomées, argiles, résines échangeuses d'ions et leurs combinaisons, et de préférence est un silicate, une zéolite ou leur combinaison, et est présent à des teneurs allant de 20 à 600 gm⁻² plus préférablement de 40 à 500 gm⁻², le plus préférablement de 100 à 400 gm⁻² par article.

20. Article selon la revendication 19, dans lequel lesdits micro-organismes produisant de l'acide lactique sont choisis parmi les genres Lactobacillus, Lactococcus, Pedioccocus, Leuconostoc, Sporolactobacillus, Bacillus ou leurs mélanges, et de préférence parmi les espèces Bacillus coagulans, Bacillus subtilis, Bacillus laterosporus, Bacillus laevolacticus, Sporolactobacillus inulinus, Lactobacillus acidophilus, Lactobacillus curvatus, Lactobacillus plantarum, Lactobacillus jenseni, Lactobacillus casei, Lactobacillus fermentum, Lactococcus lactis, Pedioccocus acidilacti, Pedioccocus pentosaceus, Pedioccocus urinae, Leuconostoc mesenteroides ou leurs mélanges.

21. Article selon l'une quelconque des revendications précédentes 19 ou 20, dans lequel les micro-organismes produisant de l'acide lactique sont des spores formant des micro-organismes produisant de l'acide lactique, et de préférence sont l'espèce B. coagulans sous sa forme vivante ou sa forme dormante (spore).

22. Article selon l'une quelconque des revendications précédentes 19 à 21, où l'article comprend plus de 10² cfu, de préférence plus de 10⁴ cfu, et plus préférablement de 10⁷ à 10¹⁰ cfu desdits micro-organismes produisant de l'acide lactique.

23. Article selon l'une quelconque des revendications précédentes 19 à 22, qui comprend des spores de micro-organismes produisant de l'acide lactique, de préférence de l'espèce Bacillus coagulans, simultanément avec tant de la silice que de la zéolite comme agent absorbant les odeurs.

24. Article selon l'une quelconque des revendications précédentes 19 à 23, qui comprend, en outre, un matériau absorbant et gélifiant, typiquement à une teneur allant de 10 gm⁻² a 300 gm⁻², de préférence de 30 gm⁻² à 150 gm⁻², plus préférablement de 55 gm⁻² à 85 gm⁻² par article.

25. Article selon l'une quelconque des revendications précédentes 19 à 24, où ledit article est un article absorbant jetable, de préférence une serviette hygiénique, un protège-slip, un tampon, une couche pour bébés, une serviette d'incontinence, une compresse pour les seins, une serviette pour la transpiration, un dispositif de gestion des déchets humains ou animaux, ou un tampon interlabial.

26. Article selon l'une quelconque des revendications précédentes 19 à 25, où ledit article est un article absorbant jetable comprenant une feuille de dessus perméable aux liquides, une feuille de fond et une âme absorbante intermédiaire entre ladite feuille de fond et ladite feuille de dessus.

27. Utilisation, dans un article absorbant jetable, en tant que moyen de régulation des odeurs, de micro-organismes produisant de l'acide lactique, de préférence des micro-organismes produisant de l'acide lactique formant des spores et plus préférablement l'espèce Bacillus coagulans, conjointement avec un absorbant des odeurs, ledit agent étant choisi dans le groupe constitué de silices, zéolites, charbons, amidons, cyclodextrine, terre à diatomées, argiles, résines échangeuses d'ions et une de leurs combinaisons et est de préférence un silicate, une zéolite ou une de leurs combinaisons et est présent à un taux allant de 20 à 600 gm⁻², plus préférablement de 40 à 500 gm⁻², le plus préférablement de 100 à 400 gm⁻² par article.
